# EUROPEAN PATENT APPLICATION

(11) **EP 4 197 554 A1**
(43) Date of publication of application: **21.06.2023**
(21) Application number: 21855635.5
(22) Date of filing: 13.08.2021
(51) Int. Cl.: A61K 39/395, A61K 31/4709, A61P 35/00

(54) **COMBINED MEDICATION FOR TREATING SOFT TISSUE SARCOMA**

(30) Priority: 13.08.2020 CN 202010814874
(71) Applicant: Chia Tai Tianqing Pharmaceutical Group Co., Ltd., Lianyungang City, Jiangsu 222062 (CN)
(72) Inventor: ZHANG, Chi, Nanjing City, Jiangsu 211100 (CN); SU, Nan, Nanjing City, Jiangsu 211100 (CN)
(74) Representative: v. Bezold & Partner Patentanwälte - PartG mbB
(86) International application number: PCT/CN2021/112549
(87) International publication number: WO 2022/033585

(57) **Abstract**

The present application relates to the field of biomedicine, and relates to a combined medication for treating soft tissue sarcoma. The combined medication comprises an anti-PD-L1 antibody and anlotinib or a pharmaceutically acceptable salt thereof. In addition, further provided is a pharmaceutical composition comprising the anti-PD-L1 antibody and anlotinib or the pharmaceutically acceptable salt thereof, or the use of the pharmaceutical composition in preparation of a medication for treating soft tissue sarcoma.

## Description

### TECHNICAL FIELD

The present application belongs to the field of biomedicine, and particularly relates to a combined medicament for treating soft tissue sarcoma.

### BACKGROUND

Soft tissue sarcoma (STS) is a group of rare malignant tumors derived from mesenchymal cells, which can occur at any age without a significant sex-related difference, is widely distributed and has various histological manifestations. Although STS accounts for less than 1% of adult malignant tumors and 15% of childhood malignant tumors, about 50% of STS patients will develop distant metastasis or even die.

The etiology of soft tissue sarcomas is still unclear, and it is generally believed that it is caused by various factors. At present, it is known that a few genetic factors have a certain relationship with the pathogenesis of some soft tissue sarcomas. The development of STS is a process that gradually spreads from the local site to the whole body, mainly manifested as swelling and/or deep-seated mass. Soft tissue sarcoma remains localized for a longer duration in adults and some children. The most common metastatic site of STS is firstly the lung, followed by other organs such as bone and liver, and regional lymph nodes are rarely involved.

Common soft tissue sarcomas include malignant fibrous histiocytoma, fibrosarcoma, liposarcoma, leiomyosarcoma, rhabdomyosarcoma, and synovial sarcoma. Other soft tissue sarcomas further include dermatofibrosarcoma protuberan, malignant peripheral nerve sheath tumor, alveolar soft-part sarcoma, clear cell sarcoma, angiosarcoma, malignant mesenchymoma, epithelioid sarcoma, undifferentiated sarcoma, and the like. Currently, multidisciplinary multimodality therapy with a major focus on surgery is extensively adopted for the treatment of soft tissue sarcoma. Soft tissue sarcoma with low malignancy is usually treated primarily by surgery or combined radiotherapy, while chemotherapy is the primary treatment for patients with advanced soft tissue sarcoma with distant metastasis. Since STS often disseminates throughout the body and lung metastasis may occur early, surgical resection is still advocated for isolated lung metastasis, and medicaments are required for other metastases. It is particularly important to select suitable drug therapy for advanced refractory STS patients who are not suitable for surgery or resistant to commonly used chemotherapeutic drugs.

Currently, only a few chemotherapeutic drugs are effective for STS, including anthracyclines such as adriamycin, adriamycin, epirubicin and pirarubicin, and alkylating agents such as cyclophosphamide, ifosfamide and dacarbazine. The effective rate of monotherapy is 14%-30%. In the first-line chemotherapy, adriamycin and ifosfamide have the best efficacy.

Since soft tissue sarcoma is a rare heterogeneous group of tumor, the treatment is usually complicated, and the available drugs and regimens are still relatively limited; therefore, it is also necessary to further develop more novel therapeutic agents with strong specificity, specific target, low toxicity and side effects, and significant curative effect, so as to achieve the expected therapeutic effect.

### BRIEF SUMMARY

In one aspect, the present application provides a therapeutic combination for use in treating soft tissue sarcoma, which comprises: an anti-PD-L1 antibody, and anlotinib or a pharmaceutically acceptable salt thereof.

In some embodiments, provided is a therapeutic combination for use in treating advanced soft tissue sarcoma, which comprises: an anti-PD-L1 antibody, and anlotinib or a pharmaceutically acceptable salt thereof.

In some embodiments, provided is a therapeutic combination for use in treating alveolar soft-part sarcoma, which comprises: an anti-PD-L1 antibody, and anlotinib or a pharmaceutically acceptable salt thereof.

In another aspect, the present application provides use of a therapeutic combination comprising an anti-PD-L1 antibody, and anlotinib or a pharmaceutically acceptable salt thereof in preparing a medicament for treating soft tissue sarcoma.

In some embodiments, provided is use of a therapeutic combination comprising an anti-PD-L1 antibody, and anlotinib or a pharmaceutically acceptable salt thereof in preparing a medicament for treating advanced soft tissue sarcoma.

In some embodiments, provided is use of a therapeutic combination comprising an anti-PD-L1 antibody, and anlotinib or a pharmaceutically acceptable salt thereof in preparing a medicament for treating alveolar soft-part sarcoma.

In yet another aspect, the present application provides use of a therapeutic combination comprising an anti-PD-L1 antibody, and anlotinib or a pharmaceutically acceptable salt thereof in treating soft tissue sarcoma.

In some embodiments, provided is use of a therapeutic combination comprising an anti-PD-L1 antibody, and anlotinib or a pharmaceutically acceptable salt thereof in treating advanced soft tissue sarcoma.

In some embodiments, provided is use of a therapeutic combination comprising an anti-PD-L1 antibody, and anlotinib or a pharmaceutically acceptable salt thereof in treating alveolar soft-part sarcoma. In some embodiments, provided is a therapeutic combination comprising an anti-PD-L1 antibody, and anlotinib or a pharmaceutically acceptable salt thereof for use in treating soft tissue sarcoma, advanced soft tissue sarcoma, or alveolar soft-part sarcoma.

In some embodiments, the therapeutic combination further comprises a pharmaceutically acceptable carrier.

In yet another aspect, the present application further provides a method for treating soft tissue sarcoma, which comprises administering to a patient in need thereof a therapeutically effective amount of an anti-PD-L1 antibody, and anlotinib or a pharmaceutically acceptable salt thereof.

In some embodiments, provided is a method for treating advanced soft tissue sarcoma, which comprises administering to a patient in need thereof a therapeutically effective amount of an anti-PD-L1 antibody, and anlotinib or a pharmaceutically acceptable salt thereof.

In some embodiments, provided is a method for treating alveolar soft-part sarcoma, which comprises administering to a patient in need thereof a therapeutically effective amount of an anti-PD-L1 antibody, and anlotinib or a pharmaceutically acceptable salt thereof.

In some embodiments, the anti-PD-L1 antibody and anlotinib or the pharmaceutically acceptable salt thereof can be administered simultaneously, nonsimultaneously, or sequentially.

In yet another aspect, the present application provides a kit for treating soft tissue sarcoma, which comprises: an anti-PD-L1 antibody, and anlotinib or a pharmaceutically acceptable salt thereof. In some embodiments, the anti-PD-L1 antibody is contained in a first compartment, and anlotinib or the pharmaceutically acceptable salt thereof is contained in a second compartment; they can be administered simultaneously, nonsimultaneously, or sequentially to a patient in need. In some embodiments, the kit further comprises instructions for combined use of an anti-PD-L1 antibody, and anlotinib or a pharmaceutically acceptable salt thereof in treating soft tissue sarcoma. In some embodiments, the kit comprises: a pharmaceutical composition comprising the anti-PD-L1 antibody, and a pharmaceutical composition comprising anlotinib or the pharmaceutically acceptable salt thereof.

In some embodiments, the present application provides a kit for treating advanced soft tissue sarcoma, which comprises: an anti-PD-L1 antibody, and anlotinib or a pharmaceutically acceptable salt thereof. In some embodiments, the anti-PD-L1 antibody is contained in a first compartment, and anlotinib or the pharmaceutically acceptable salt thereof is contained in a second compartment; they can be administered simultaneously, nonsimultaneously, or sequentially to a patient in need. In some embodiments, the kit further comprises instructions for combined use of an anti-PD-L1 antibody, and anlotinib or a pharmaceutically acceptable salt thereof in treating advanced soft tissue sarcoma. In some embodiments, the kit comprises: a pharmaceutical composition comprising the anti-PD-L1 antibody, and a pharmaceutical composition comprising anlotinib or the pharmaceutically acceptable salt thereof.

In some embodiments, the present application provides a kit for treating alveolar soft-part sarcoma, which comprises: an anti-PD-L1 antibody, and anlotinib or a pharmaceutically acceptable salt thereof. In some embodiments, the anti-PD-L1 antibody is contained in a first compartment, and anlotinib or the pharmaceutically acceptable salt thereof is contained in a second compartment; they can be administered simultaneously, nonsimultaneously, or sequentially to a patient in need. In some embodiments, the kit further comprises instructions for combined use of an anti-PD-L1 antibody, and anlotinib or a pharmaceutically acceptable salt thereof in treating alveolar soft-part sarcoma. In some embodiments, the kit comprises: a pharmaceutical composition comprising the anti-PD-L1 antibody, and a pharmaceutical composition comprising anlotinib or the pharmaceutically acceptable salt thereof.

In some embodiments, the therapeutic combination described herein comprises: an anti-PD-L1 humanized monoclonal antibody, and anlotinib or a pharmaceutically acceptable salt thereof.

In some embodiments, the therapeutic combination comprising an anti-PD-L1 antibody, and anlotinib or a pharmaceutically acceptable salt thereof described herein comprises: a pharmaceutical composition comprising the anti-PD-L1 antibody, and a pharmaceutical composition comprising anlotinib or the pharmaceutically acceptable salt thereof.

In some embodiments, the pharmaceutical composition further comprises a pharmaceutically acceptable carrier.

In some embodiments, the therapeutic combination comprising an anti-PD-L1 antibody, and anlotinib or a pharmaceutically acceptable salt thereof described herein comprises: a pharmaceutical composition comprising 600-2400 mg of the anti-PD-L1 antibody. In some embodiments, the anti-PD-L1 antibody is in the form of a pharmaceutical composition at a dose of 600-2400 mg or 600 mg, 800 mg, 1000 mg, 1200 mg, 1400 mg, 1600 mg, 1800 mg, 2000 mg, 2200 mg, or 2400 mg. In some embodiments, the therapeutic combination comprises: a pharmaceutical composition comprising 600-2400 mg of an anti-PD-L1 antibody, wherein the pharmaceutical composition comprising the anti-PD-L1 antibody is in a unit dose or in multiple doses.

In some embodiments, the therapeutic combination comprising an anti-PD-L1 antibody, and anlotinib or a pharmaceutically acceptable salt thereof described herein comprises: a pharmaceutical composition of 6-12 mg of anlotinib or a pharmaceutically acceptable salt thereof. In some embodiments, the therapeutic combination comprises: a pharmaceutical composition of anlotinib or a pharmaceutically acceptable salt thereof at a unit dose of 6 mg, 8 mg, 10 mg and/or 12 mg. In some embodiments, anlotinib or the pharmaceutically acceptable salt thereof is in the form of a pharmaceutical composition at a dose of 6-12 mg or 6 mg, 8 mg, 10 mg or 12 mg.

In some embodiments, the therapeutic combination comprising the anti-PD-L1 antibody, and anlotinib or the pharmaceutically acceptable salt thereof described herein comprises: a pharmaceutical composition of 600-2400 mg of the anti-PD-L1 antibody and a pharmaceutical composition of 6 mg, 8 mg, 10 mg and/or 12 mg of anlotinib or the pharmaceutically acceptable salt thereof in a unit dose, wherein the pharmaceutical composition of the anti-PD-L1 antibody is in a unit dose or in multiple doses; in some embodiments, the therapeutic combination comprises: a pharmaceutical composition of 600-2400 mg of the anti-PD-L1 antibody provided in multiple-dose form and a pharmaceutical composition of 6 mg, 8 mg, 10 mg and/or 12 mg of anlotinib or the pharmaceutically acceptable salt thereof in a unit dose; in some embodiments, the therapeutic combination is a formulation suitable for administration within a single treatment cycle (e.g., a 21-day treatment cycle) and comprises a pharmaceutical composition of 600-2400 mg of the anti-PD-L1 antibody and a pharmaceutical composition of 84-168 mg of anlotinib or the pharmaceutically acceptable salt thereof.

In some embodiments, the therapeutic combination comprising an anti-PD-L1 antibody, and anlotinib or a pharmaceutically acceptable salt thereof described herein comprises: the anti-PD-L1 antibody and anlotinib or the pharmaceutically acceptable salt thereof at a weight ratio of (0.35-29):1, (3.5-29):1, (3.5-14.5):1, or (7-14.5):1. The anti-PD-L1 antibody and anlotinib or the pharmaceutically acceptable salt thereof can be separately packaged or packaged together. Anlotinib can be packaged in multiple aliquots (e.g., 2 aliquots, 7 aliquots, 14 aliquots, 28 aliquots or more); the anti-PD-L1 antibody can be packaged in a single aliquot or in multiple aliquots (e.g., 2 aliquots, 4 aliquots or more).

In some embodiments, the present application provides a therapeutic combination comprising an anti-PD-L1 antibody, and anlotinib or a pharmaceutically acceptable salt thereof, wherein the anti-PD-L1 antibody and anlotinib or the pharmaceutically acceptable salt thereof are each in the form of a pharmaceutical composition and can be administered simultaneously, nonsimultaneously, or sequentially.

The amount of the anti-PD-L1 antibody administered may be determined according to the severity of the disease, the response of the disease, any treatment-related toxicity, the age and health of a patient, and the like. For example, the anti-PD-L1 antibody may be administered at a daily dose of 600-2400 mg. In some embodiments, the anti-PD-L1 antibody may be administered at a daily dose of 600 mg, 800 mg, 1000 mg, 1200 mg, 1400 mg, 1600 mg, 1800 mg, 2000 mg, 2200 mg, or 2400 mg. In some embodiments, the anti-PD-L1 antibody is administered parenterally. In some embodiments, the anti-PD-L1 antibody is administered intravenously; in some embodiments, the pharmaceutical composition of the anti-PD-L1 antibody is at a concentration of 10-60 mg/mL. In some embodiments, the pharmaceutical composition of the anti-PD-L1 antibody is at a concentration of 10 mg/mL, 20 mg/mL, 30 mg/mL, 40 mg/mL, 50 mg/mL, or 60 mg/mL.

In some embodiments, the anti-PD-L1 antibody is administered once every 1 week, every 2 weeks, every 3 weeks, or every 4 weeks. In some embodiments, the anti-PD-L1 antibody is administered at a dose of 600-2400 mg each time. In some embodiments, the anti-PD-L1 antibody is administered once every 3 weeks at a dose of 600-2400 mg each time. In some embodiments, anlotinib or the pharmaceutically acceptable salt thereof is administered at a daily dose of 6 mg, 8 mg, 10 mg, or 12 mg on a regimen of consecutively 2-week treatment and then 1-week interruption. In some embodiments, anlotinib or the pharmaceutically acceptable salt thereof is administered once daily at a daily dose of 6 mg, 8 mg, 10 mg, or 12 mg on a regimen of consecutively 2-week treatment and then 1-week interruption.

In some embodiments, the therapeutic combination comprising an anti-PD-L1 antibody, and anlotinib or a pharmaceutically acceptable salt thereof described herein comprises a pharmaceutical composition comprising the anti-PD-L1 antibody and a pharmaceutical composition comprising anlotinib or the pharmaceutically acceptable salt thereof, wherein the anti-PD-L1 antibody is prepared in a unit dose or in multiple doses suitable for providing 600-2400 mg of the anti-PD-L1 antibody for a patient at first administration, and the pharmaceutical composition comprising anlotinib or the pharmaceutically acceptable salt thereof is prepared in a unit dose suitable for providing 6 mg, 8 mg, 10 mg and/or 12 mg of anlotinib or the pharmaceutically acceptable salt thereof for a patient daily for 14 consecutive days.

In some embodiments, the therapeutic combination comprising an anti-PD-L1 antibody, and anlotinib or a pharmaceutically acceptable salt thereof described herein comprises: a pharmaceutical composition comprising the anti-PD-L1 antibody in which the anti-PD-L1 antibody is at a concentration of 10-60 mg/mL, and a pharmaceutical composition comprising 6 mg, 8 mg, 10 mg and/or 12 mg of anlotinib or the pharmaceutically acceptable salt thereof in a unit dose.

In some embodiments, the therapeutic combination comprising an anti-PD-L1 antibody, and anlotinib or a pharmaceutically acceptable salt thereof described herein comprises: a pharmaceutical composition comprising the anti-PD-L1 antibody in which the anti-PD-L1 antibody is at a concentration of 30 mg/mL, and a pharmaceutical composition comprising 8 mg, 10 mg and/or 12 mg of anlotinib or the pharmaceutically acceptable salt thereof in a unit dose.

In some embodiments, the therapeutic combination comprising an anti-PD-L1 antibody, and anlotinib or a pharmaceutically acceptable salt thereof described herein comprises: a pharmaceutical composition comprising the anti-PD-L1 antibody in which the anti-PD-L1 antibody is at a concentration of 10 mg/mL, and a pharmaceutical composition comprising 8 mg, 10 mg and/or 12 mg of anlotinib or the pharmaceutically acceptable salt thereof in a unit dose.

In some embodiments, the therapeutic combination comprising an anti-PD-L1 antibody, and anlotinib or a pharmaceutically acceptable salt thereof described herein comprises: a pharmaceutical composition comprising 1200 mg of the anti-PD-L1 antibody provided in multiple-dose form, and a pharmaceutical composition comprising 8 mg, 10 mg and/or 12 mg of anlotinib or the pharmaceutically acceptable salt thereof in a unit dose. In some embodiments, the kit is suitable for administration within a single treatment cycle (e.g., a treatment cycle of 21 days), and comprises a pharmaceutical composition comprising 600-2400 mg of the anti-PD-L1 antibody and a pharmaceutical composition comprising 84-168 mg of anlotinib or the pharmaceutically acceptable salt thereof.

In some embodiments, provided is a method for treating soft tissue sarcoma, which comprises administering to a patient in need thereof a therapeutically effective amount of an anti-PD-L1 antibody, and anlotinib or a pharmaceutically acceptable salt thereof. In some embodiments, the anti-PD-L1 antibody and anlotinib or a pharmaceutically acceptable salt thereof are administered simultaneously, nonsimultaneously, or sequentially. In some embodiments, the anti-PD-L1 antibody is administered once every 1 week, every 2 weeks, every 3 weeks, or every 4 weeks; in some embodiments, the anti-PD-L1 antibody is administered at a dose of 600-2400 mg each time. In some embodiments, the anti-PD-L1 antibody is administered at a single dose of 600 mg, 800 mg, 1000 mg, 1200 mg, 1400 mg, 1600 mg, 1800 mg, 2000 mg, 2200 mg, or 2400 mg. In some embodiments, anlotinib or the pharmaceutically acceptable salt thereof is administered at a daily dose of 6-12 mg. In some embodiments, anlotinib or the pharmaceutically acceptable salt thereof is administered at a daily dose of 8-12 mg. In some embodiments, anlotinib or the pharmaceutically acceptable salt thereof is administered at a daily dose of 6 mg, 8 mg, 10 mg, or 12 mg. In some embodiments, the anlotinib or the pharmaceutically acceptable salt thereof is administered at a dose of 6 mg, 8 mg, 10 mg, or 12 mg once daily. In some embodiments, anlotinib or the pharmaceutically acceptable salt thereof is administered on a regimen of consecutively 2-week treatment and then 1-week interruption. In some embodiments, every 3 weeks are counted as one treatment cycle. In some embodiments, 21 days are counted as one treatment cycle, and the anti-PD-L1 antibody is administered to the patient on the first day of each treatment cycle. In some embodiments, every 3 weeks are counted as one treatment cycle, the anti-PD-L1 antibody is administered on the first day of each cycle, and anlotinib or the pharmaceutically acceptable salt thereof is administered on days 1-14 of each cycle. In some embodiments, the anti-PD-L1 antibody is administered parenterally. In some embodiments, the pharmaceutical composition of the anti-PD-L1 antibody is at a concentration of 10-60 mg/mL. In some embodiments, the pharmaceutical composition of the anti-PD-L1 antibody is at a concentration of 10 mg/mL, 20 mg/mL, 30 mg/mL, 40 mg/mL, 50 mg/mL, or 60 mg/mL. In some embodiments, anlotinib or the pharmaceutically acceptable salt thereof is administered orally.

The regimen for the anti-PD-L1 antibody can be determined comprehensively depending on the activity and toxicity of the medicament, tolerance of a patient, and the like. In some embodiments, for the anti-PD-L1 antibody, every 1 week, every 2 weeks, every 3 weeks, or every 4 weeks are counted as one treatment cycle. In some embodiments, the anti-PD-L1 antibody is administered once a week, every 2 weeks, every 3 weeks, or every 4 weeks.

In some embodiments, the anti-PD-L1 antibody is administered at a dose of 600-2400 mg per treatment cycle. In some embodiments, the anti-PD-L1 antibody is administered at a dose of 1200 mg per treatment cycle. Anlotinib or a pharmaceutically acceptable salt thereof

The chemical name of anlotinib is 1-[[[4-(4-fluoro-2-methyl-1*H*-indol-5-yl)oxy-6-methoxyquinolin-7-yl]oxy]methyl]cyclopropylamine, which has the following structural formula:

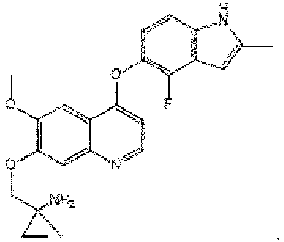

The pharmaceutically acceptable salts of anlotinib include, but are not limited to, salts formed from anlotinib and acids selected from the group consisting of the following: hydrochloric acid, hydrobromic acid, sulfuric acid, nitric acid, phosphoric acid, acetic acid, trifluoroacetic acid, propionic acid, hexanoic acid, heptanoic acid, cyclopentanepropionic acid, glycolic acid, pyruvic acid, lactic acid, malonic acid, succinic acid, malic acid, maleic acid, fumaric acid, tartaric acid, citric acid, benzoic acid, cinnamic acid, mandelic acid, methanesulfonic acid, ethanesulfonic acid, 1,2-ethanedisulfonic acid, 2-hydroxyethanesulfonic acid, benzenesulfonic acid, p-chlorobenzenesulfonic acid, p-toluenesulfonic acid, 3-phenylpropionic acid, trimethylacetic acid, t-butylacetic acid, dodecyl sulfuric acid, gluconic acid, glutamic acid, hydroxynaphthoic acid, salicylic acid and stearic acid; in some embodiments, the pharmaceutically acceptable salt is a hydrochloride or a maleate; in some embodiments, the pharmaceutically acceptable salt is a dihydrochloride.

Unless otherwise stated, the dose of anlotinib or the pharmaceutically acceptable salt thereof referred to in the present application is based on the molecular weight of the free base of anlotinib.

Anlotinib or the pharmaceutically acceptable salt thereof may be administered through various routes including gastrointestinal and parenteral including, but not limited to, oral, intravenous, intra-arterial, transdermal, sublingual, intramuscular, rectal, transbuccal, intranasal, inhalational, vaginal, intraocular, local, subcutaneous, intra-adipose, intra-articular, intraperitoneal, and intrathecal administrations. In some specific embodiments, the drug is administered orally. The amount of anlotinib or the pharmaceutically acceptable salt thereof administered can be determined according to the severity of the disease, the response of the disease, any treatment-related toxicity, and the age and health of a patient. For example, the daily dose for administering anlotinib or the pharmaceutically acceptable salt thereof may be from 2-20 mg. In some embodiments, the daily dose for administering anlotinib or the pharmaceutically acceptable salt thereof may be 2 mg, 3 mg, 4 mg, 5 mg, 6 mg, 7 mg, 8 mg, 9 mg, 10 mg, 11 mg, 12 mg, 13 mg, 14 mg, 15 mg, and 16 mg. Anlotinib or the pharmaceutically acceptable salt thereof can be administered once or multiple times daily. In some embodiments, anlotinib or the pharmaceutically acceptable salt thereof is administered once daily in the form of an oral solid formulation.

The administration regimen of anlotinib or the pharmaceutically acceptable salt thereof can be determined depending on the combination of the activity and toxicity of the drug, and the tolerance of a patient, and the like. Preferably, anlotinib or the pharmaceutically acceptable salt thereof is administered at intervals. The interval administration includes a treatment period and an interruption period. Anlotinib or the pharmaceutically acceptable salt thereof can be administered once or multiple times daily in the treatment period. For example, the ratio of the treatment period to the interruption period in days is 2:(0.5-5), 2:(0.5-3), 2:(0.5-2), or 2:(0.5-1). In some embodiments, anlotinib or the pharmaceutically acceptable salt thereof is administered on a regimen of consecutively 2-week treatment and then 2-week interruption. In some embodiments, anlotinib or the pharmaceutically acceptable salt thereof is administered on a regimen of consecutively 2-week treatment and then 1-week interruption. In some embodiments, anlotinib or the pharmaceutically acceptable salt thereof is administered on a regimen of consecutively 5-day treatment and then 2-day interruption. For example, anlotinib or the pharmaceutically acceptable salt thereof can be orally administered at a dose of 6 mg, 8 mg, 10 mg or 12 mg once daily on a regimen of consecutively 2-week treatment and then 1-week interruption.

### Pharmaceutical composition of anlotinib or the pharmaceutically acceptable salt thereof

In some embodiments of the present application, a unit dose of the pharmaceutical composition of anlotinib or the pharmaceutically acceptable salt thereof comprises 6 mg, 8 mg, 10 mg or 12 mg of anlotinib or a pharmaceutically acceptable salt thereof. In some embodiments, the pharmaceutical composition of anlotinib or a pharmaceutically acceptable salt thereof is in the form of an oral solid formulation.

In some embodiments of the present application, according to the regimen of 2-week treatment and then 1-week interruption, every 3 weeks are counted as one treatment cycle, and a total dose of anlotinib or the pharmaceutically acceptable salt thereof administered per treatment cycle is 84-168 mg. In some embodiments, a total dose of anlotinib or the pharmaceutically acceptable salt thereof includes an amount selected from the group consisting of 84 mg, 112 mg, 140 mg and 168 mg or from a range formed of any of the aforementioned values. In some embodiments, a total dose of anlotinib or the pharmaceutically acceptable salt thereof includes 112-168 mg. In some embodiments, the pharmaceutical composition includes, but is not limited to, a formulation suitable for oral, parenteral and local administration. In some embodiments, the pharmaceutical composition is a formulation suitable for oral administration. In some embodiments, the pharmaceutical composition is a solid formulation suitable for oral administration. In some embodiments, the pharmaceutical composition includes, but is not limited to, tablets and capsules.

### Anti-PD-L1 antibody

In some embodiments of the present application, the anti-PD-L1 antibody is one or more of the antibodies described in CN107001463A.

In some embodiments of the present application, the anti-PD-L1 antibody is an isolated anti-PD-L1 antibody. In some embodiments of the present application, the anti-PD-L1 antibody is an anti-PD-L1 humanized monoclonal antibody. In some embodiments of the present application, the anti-PD-L1 antibody is an isolated anti-PD-L1 humanized monoclonal antibody. In some embodiments of the present application, the anti-PD-L1 antibody may be an IgG1 or IgG4 antibody. In some embodiments of the present application, the anti-PD-L1 antibody is an IgG1 antibody. In some embodiments, the anti-PD-L1 antibody is a glycosylated IgG1 antibody.

In some embodiments of the present application, the anti-PD-L1 antibody comprises the following amino acid sequences: a heavy chain CDR1 region having at least 80% (e.g., 81%, 82%, 83%, 84%, 85%, 86%, 87%, 88%, 89%, 90%, 91%, 92%, 93%, 94%, 95%, 96%, 97%, 98%, 99% or 100%) homology to an amino acid sequence set forth in SEQ ID NO: 1 or SEQ ID NO: 4; a heavy chain CDR2 region having at least 80% (e.g., 81%, 82%, 83%, 84%, 85%, 86%, 87%, 88%, 89%, 90%, 91%, 92%, 93%, 94%, 95%, 96%, 97%, 98%, 99% or 100%) homology to an amino acid sequence set forth in SEQ ID NO: 2 or SEQ ID NO: 5; a heavy chain CDR3 region having at least 80% (e.g., 81%, 82%, 83%, 84%, 85%, 86%, 87%, 88%, 89%, 90%, 91%, 92%, 93%, 94%, 95%, 96%, 97%, 98%, 99% or 100%) homology to an amino acid sequence set forth in SEQ ID NO: 3 or SEQ ID NO: 6; a light chain CDR1 region having at least 80% (e.g., 81%, 82%, 83%, 84%, 85%, 86%, 87%, 88%, 89%, 90%, 91%, 92%, 93%, 94%, 95%, 96%, 97%, 98%, 99% or 100%) homology to an amino acid sequence set forth in SEQ ID NO: 7 or SEQ ID NO: 10; a light chain CDR2 region having at least 80% (e.g., 81%, 82%, 83%, 84%, 85%, 86%, 87%, 88%, 89%, 90%, 91%, 92%, 93%, 94%, 95%, 96%, 97%, 98%, 99% or 100%) homology to an amino acid sequence set forth in SEQ ID NO: 8 or SEQ ID NO: 11; and a light chain CDR3 region having at least 80% (e.g., 81%, 82%, 83%, 84%, 85%, 86%, 87%, 88%, 89%, 90%, 91%, 92%, 93%, 94%, 95%, 96%, 97%, 98%, 99% or 100%) homology to an amino acid sequence set forth in SEQ ID NO: 9 or SEQ ID NO: 12.

In some embodiments, the anti-PD-L1 antibody comprises the following amino acid sequences:In some embodiments, the anti-PD-L1 antibody comprises the following amino acid sequences: a heavy chain CDR1 region having at least 80% homology to an amino acid sequence set forth in SEQ ID NO: 1 or SEQ ID NO: 4; or a heavy chain CDR2 region having at least 80% homology to an amino acid sequence set forth in SEQ ID NO: 2 or SEQ ID NO: 5; or a heavy chain CDR3 region having at least 80% homology to an amino acid sequence set forth in SEQ ID NO: 3 or SEQ ID NO: 6; or a light chain CDR1 region having at least 80% homology to an amino acid sequence set forth in SEQ ID NO: 7 or SEQ ID NO: 10; or a light chain CDR2 region having at least 80% homology to an amino acid sequence set forth in SEQ ID NO: 8 or SEQ ID NO: 11; or a light chain CDR3 region having at least 80% homology to an amino acid sequence set forth in SEQ ID NO: 9 or SEQ ID NO: 12.

In some embodiments, the anti-PD-L1 antibody comprises the following amino acid sequences: a heavy chain CDR1 region having at least 80% homology to an amino acid sequence set forth in SEQ ID NO: 1 or SEQ ID NO: 4; a heavy chain CDR2 region having at least 80% homology to an amino acid sequence set forth in SEQ ID NO: 2 or SEQ ID NO: 5; a heavy chain CDR3 region having at least 80% homology to an amino acid sequence set forth in SEQ ID NO: 3 or SEQ ID NO: 6; a light chain CDR1 region having at least 80% homology to an amino acid sequence set forth in SEQ ID NO: 7 or SEQ ID NO: 10; a light chain CDR2 region having at least 80% homology to an amino acid sequence set forth in SEQ ID NO: 8 or SEQ ID NO: 11; and a light chain CDR3 region having at least 80% homology to an amino acid sequence set forth in SEQ ID NO: 9 or SEQ ID NO: 12.

In some embodiments of the present application, the anti-PD-L1 antibody comprises the following amino acid sequences: a heavy chain CDR1 region selected from the group consisting of SEQ ID NO: 1 and SEQ ID NO: 4; a heavy chain CDR2 region selected from the group consisting of SEQ ID NO: 2 and SEQ ID NO: 5; a heavy chain CDR3 region selected from the group consisting of SEQ ID NO: 3 and SEQ ID NO: 6; a light chain CDR1 region selected from the group consisting of SEQ ID NO: 7 and SEQ ID NO: 10; a light chain CDR2 region selected from the group consisting of SEQ ID NO: 8 and SEQ ID NO: 11; and a light chain CDR3 region selected from the group consisting of SEQ ID NO: 9 and SEQ ID NO: 12.

In some embodiments of the present application, an isolated anti-PD-L1 antibody described herein comprises: a heavy chain CDR1 region having an amino acid sequence set forth in SEQ ID NO: 1; a heavy chain CDR2 region having an amino acid sequence set forth in SEQ ID NO: 2; a heavy chain CDR3 region having an amino acid sequence set forth in SEQ ID NO: 3; a light chain CDR1 region having an amino acid sequence set forth in SEQ ID NO: 7; a light chain CDR2 region having an amino acid sequence set forth in SEQ ID NO: 8; and a light chain CDR3 region having an amino acid sequence set forth in SEQ ID NO: 9.

Each of the CDR regions described herein and the variants thereof described above are capable of specifically recognizing and binding to PD-L1, thereby effectively blocking the signaling between PD-L1 and PD-1.

In some embodiments of the present application, the anti-PD-L1 antibody comprises the following amino acid sequences: a heavy chain variable region having at least 80% (e.g., 81%, 82%, 83%, 84%, 85%, 86%, 87%, 88%, 89%, 90%, 91%, 92%, 93%, 94%, 95%, 96%, 97%, 98%, 99% or 100%) homology to an amino acid sequence set forth in SEQ ID NO: 13 or SEQ ID NO: 14; and a light chain variable region having at least 80% (e.g., 81%, 82%, 83%, 84%, 85%, 86%, 87%, 88%, 89%, 90%, 91%, 92%, 93%, 94%, 95%, 96%, 97%, 98%, 99% or 100%) homology to an amino acid sequence set forth in SEQ ID NO: 15 or SEQ ID NO: 16.

In some embodiments of the present application, the anti-PD-L1 antibody comprises the following amino acid sequences: a heavy chain variable region set forth in SEQ ID NO: 13, and a light chain variable region set forth in SEQ ID NO: 15.

In some embodiments of the present application, the anti-PD-L1 antibody comprises the following amino acid sequences: a heavy chain variable region set forth in SEQ ID NO: 14, and a light chain variable region set forth in SEQ ID NO: 16.

In some embodiments of the present application, the anti-PD-L1 antibody comprises the following amino acid sequences: a heavy chain amino acid sequence set forth in SEQ ID NO: 17, and a light chain amino acid sequence set forth in SEQ ID NO: 18.

In some embodiments of the present application, the anti-PD-L1 antibody comprises the following amino acid sequences: a heavy chain amino acid sequence set forth in SEQ ID NO: 19, and a light chain amino acid sequence set forth in SEQ ID NO: 20.

In some embodiments of the present application, the anti-PD-L1 antibody comprises the following amino acid sequences: a heavy chain amino acid sequence set forth in SEQ ID NO: 21, and a light chain amino acid sequence set forth in SEQ ID NO: 18.

In one specific embodiment, the anti-PD-L1 humanized mAb provided herein comprises one or more conservatively substituted variants selected from the group consisting of: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20 and SEQ ID NO: 21. The humanized anti-PD-L1 mAb comprising the conservatively substituted variants retains the ability to specifically recognize and bind to PD-L1.

In some embodiments of the present application, the anti-PD-L1 antibody comprises heavy chain complementarity determining regions (CDRs) selected from the group consisting of heavy chain CDRs derived from antibodies 13C5 and 5G11, and light chain CDRs selected from the group consisting of light chain CDRs derived from antibodies 13C5 and 5G11. In one embodiment, the anti-PD-L1 antibody described herein comprises: a heavy chain variable region selected from the group consisting of heavy chain variable regions of chimeric antibodies ch5G11-hIgG1, ch5G11-hIgG4, ch13C5-hIgG1, and ch13C5-hIgG4; and a light chain variable region selected from the group consisting of light chain variable regions of chimeric antibodies ch5G11-hIgG1, ch5G11-hIgG4, ch13C5-hIgG1, and ch13C5-hIgG4. In one embodiment, the anti-PD-L1 antibody described herein comprises: a heavy chain variable region selected from the group consisting of heavy chain variable regions of humanized antibodies hu13C5-hIgG1, hu13C5-hIgG4, hu5G11-hIgG1, and hu5G11-hIgG4; and a light chain variable region selected from the group consisting of light chain variable regions of humanized antibodies hu13 C5-hIgG 1, hu13C5-hIgG4, hu5G11-hIgG1, and hu5G11-hIgG4. Reference can be made to the description of the patents WO2016022630 or CN107001463A: 13C5, ch13C5-hIgG1, ch13C5-hIgG4, hu13C5-hIgG1 or hu13C5-hIgG4 comprises an HCDR1 sequence of SYGMS (SEQ ID NO: 4), an HCDR2 sequence of SISSGGSTYYPDSVKG (SEQ ID NO: 5), an HCDR3 sequence of GYDSGFAY (SEQ ID NO: 6), an LCDR1 sequence of ASQSVSTSSSSFMH (SEQ ID NO: 10), an LCDR2 sequence of YASNLES (SEQ ID NO: 11), and an LCDR3 sequence of QHSWEIPYT (SEQ ID NO: 12); 5G11, ch5G11-hIgG1, ch5G11-hIgG4, hu5G11-hIgG1 or hu5G11-hIgG4 comprises an HCDR1 sequence of TYGVH (SEQ ID NO: 1), an HCDR2 sequence of VIWRGVTTDYNAAFMS (SEQ ID NO: 2), an HCDR3 sequence of LGFYAMDY (SEQ ID NO: 3), an LCDR1 sequence of KASQSVSNDVA (SEQ ID NO: 7), an LCDR2 sequence of YAANRYT (SEQ ID NO: 8), and an LCDR3 sequence of QQDYTSPYT (SEQ ID NO: 9). In one embodiment, hu5G11-hIgG1 has a heavy chain amino acid sequence set forth in SEQ ID NO: 17, and a light chain amino acid sequence set forth in SEQ ID NO: 18.

In some embodiments of the present application, the anti-PD-L1 antibody in the therapeutic combination may be selected from one or more. As used herein, the term "more" refers to more than one, for example, two, three, four, five or more. For example, in some embodiments of the present application, the anti-PD-L1 antibody comprises a heavy chain variable region set forth in SEQ ID NO: 13 and a light chain variable region set forth in SEQ ID NO: 15, or comprises a heavy chain variable region set forth in SEQ ID NO: 14 and a light chain variable region set forth in SEQ ID NO: 16, or comprises a combination thereof. As another example, the anti-PD-L1 antibody comprises a heavy chain amino acid sequence set forth in SEQ ID NO: 17 and a light chain amino acid sequence set forth in SEQ ID NO: 18, or comprises a heavy chain amino acid sequence set forth in SEQ ID NO: 19 and a light chain amino acid sequence set forth in SEQ ID NO: 20, or comprises a heavy chain amino acid sequence set forth in SEQ ID NO: 21 and a light chain amino acid sequence set forth in SEQ ID NO: 18, or comprises combinations of any of the foregoing.

### Pharmaceutical composition of anti-PD-L1 antibody

In some embodiments of the present application, the pharmaceutical composition of the anti-PD-L1 antibody comprises 600-2400 mg of the anti-PD-L1 antibody. In some embodiments, the pharmaceutical composition of the anti-PD-L1 antibody comprises 600 mg, 900 mg, 1200 mg, 1500 mg, 1800 mg, 2100 mg and 2400 mg or a range formed of any of the aforementioned values of the anti-PD-L1 antibody. In some embodiments, the pharmaceutical composition of the anti-PD-L1 antibody comprises 600-2100 mg or 900-1500 mg of the anti-PD-L1 antibody; wherein the pharmaceutical composition of the anti-PD-L1 antibody may be present in multiple-dose or in unit-dose form.

In some embodiments of the present application, the pharmaceutical composition of the anti-PD-L1 antibody comprises 300 mg, 600 mg, or 1200 mg of the anti-PD-L1 antibody. In some embodiments of the present application, provided is a pharmaceutical composition formulated as a unit dose of an anti-PD-L1 antibody, which comprises 300 mg, 600 mg, or 1200 mg of the anti-PD-L1 antibody.

In a specific embodiment, the pharmaceutical composition of the anti-PD-L1 antibody is a solution for injection. In some embodiments, the pharmaceutical composition of the anti-PD-L1 antibody is aqueous solution for injection. In some embodiments of the present application, the pharmaceutical composition of the anti-PD-L1 antibody comprises one or more of a buffer, an isotonicity modifier, a stabilizer and/or a surfactant. In particular, the pharmaceutical composition of the anti-PD-L1 antibody comprises 1-150 mg/mL anti-PD-L1 antibody (e.g., mAb), 3-50 mM buffer, 2-150 mg/mL isotonicity modifier/stabilizer and 0.01-0.8 mg/mL surfactant, and has a pH of 4.5-6.8.

In some embodiments of the present application, in the pharmaceutical composition of the anti-PD-L1 antibody, the anti-PD-L1 mAb is at a concentration of 5-150 mg/mL (w/v); in some embodiments, the anti-PD-L1 mAb is at a concentration of 10-60 mg/mL (w/v); in some embodiments, the anti-PD-L1 mAb is at a concentration of 10-30 mg/mL (w/v). In some embodiments, the anti-PD-L1 mAb is at a concentration of 10 mg/mL (w/v), 20 mg/mL (w/v), 30 mg/mL (w/v), 40 mg/mL (w/v), 50 mg/mL (w/v), 60 mg/mL (w/v), 70 mg/mL (w/v), 80 mg/mL (w/v), 90 mg/mL (w/v), 100 mg/mL (w/v), 110 mg/mL (w/v), or 120 mg/mL (w/v); in some embodiments, the anti-PD-L1 mAb is at a concentration of 10 mg/mL (w/v), 20 mg/mL (w/v), 30 mg/mL (w/v), 40 mg/mL (w/v), 50 mg/mL (w/v), or 60 mg/mL (w/v); in some embodiments, the anti-PD-L1 mAb is at a concentration of 10 mg/mL (w/v), 20 mg/mL (w/v), or 30 mg/mL (w/v). In some embodiments, the anti-PD-L1 mAb is at a concentration of 10 mg/mL (w/v). In other embodiments, the anti-PD-L1 mAb is at a concentration of 30 mg/mL (w/v). In other embodiments, the anti-PD-L1 mAb is at a concentration of 60 mg/mL (w/v).

In some embodiments of the present application, the buffer is a histidine salt buffer. Preferably, the histidine salt buffer is at a concentration of 5-30 mM; in some embodiments, the histidine salt buffer is at a concentration of 10-25 mM; in some embodiments, the histidine salt buffer is at a concentration of 10-20 mM; in some embodiments, the histidine salt buffer is at a concentration of 10-15 mM. In some embodiments, the histidine salt buffer is at a concentration of 5 mM, 10 mM, 15 mM, 20 mM, 25 mM, or 30 mM. In some embodiments, the histidine salt buffer is at a concentration of 10 mM. In other embodiments, the histidine salt buffer is at a concentration of 15 mM. In other embodiments, the histidine salt buffer is at a concentration of 20 mM. The histidine salt buffer comprises histidine and hydrochloric acid.

In some embodiments of the present application, the isotonicity modifier/stabilizer is sucrose at 20-150 mg/mL (w/v); in some embodiment, the isotonicity modifier/stabilizer is sucrose at 40-100 mg/mL (w/v); in some embodiment, the isotonicity modifier/stabilizer is sucrose at 60-80 mg/mL (w/v). In some specific embodiments, the sucrose is at a concentration of 40 mg/mL, 50 mg/mL, 60 mg/mL, 70 mg/mL, 80 mg/mL, 90 mg/mL or 100 mg/mL. In some specific embodiments, the sucrose is at a concentration of 60 mg/mL. In some specific embodiments, the sucrose is at a concentration of 70 mg/mL. In some specific embodiments, the sucrose is at a concentration of 80 mg/mL. In some specific embodiments, the sucrose is at a concentration of 90 mg/mL.

In some embodiments of the present application, the surfactant is selected from the group consisting of polysorbate 80, polysorbate 20 and poloxamer 188; in some embodiments, the surfactant is selected from the group consisting of polysorbate 80 and polysorbate 20; in some embodiments, the surfactant is selected from the group consisting of polysorbate 80. In some embodiments, the surfactant is at a concentration of 0.05-0.6 mg/mL (w/v); in some embodiments, the surfactant is at a concentration of 0.1-0.4 mg/mL (w/v); in some embodiments, the surfactant is at a concentration of 0.2-0.3 mg/mL (w/v).

In some embodiments of the present application, the surfactant is polysorbate 80 or polysorbate 20 at 0.01-0.8 mg/mL (w/v). In some specific embodiments, the surfactant is polysorbate 80 at 0.05-0.6 mg/mL; in some embodiments, the surfactant is polysorbate 80 at 0.1-0.4 mg/mL; in some embodiments, the surfactant is polysorbate 80 at 0.2-0.3 mg/mL; in some embodiments, the surfactant is polysorbate 80 at 0.2 mg/mL. In some embodiments, the pharmaceutical composition has a polysorbate 80 content of 0.1 mg/mL, 0.2 mg/mL, 0.3 mg/mL, 0.4 mg/mL, 0.5 mg/mL, or 0.6 mg/mL. In some embodiments, the pharmaceutical composition has a polysorbate 80 content of 0.2 mg/mL, 0.3 mg/mL, 0.4 mg/mL, or 0.5 mg/mL. In some embodiments, the pharmaceutical composition has a polysorbate 80 content of 0.2 mg/mL, 0.3 mg/mL, or 0.4 mg/mL. In some embodiments, the pharmaceutical composition has a polysorbate 80 content of 0.2 mg/mL. In some embodiments, the pharmaceutical composition has a polysorbate 80 content of 0.1 mg/mL. In other embodiments, the pharmaceutical composition has a polysorbate 80 content of 0.2 mg/mL. In some embodiments, the pharmaceutical composition has a polysorbate 80 content of 0.3 mg/mL. In other embodiments, the pharmaceutical composition has a polysorbate 80 content of 0.4 mg/mL. In some embodiments, the pharmaceutical composition has a polysorbate 80 content of 0.5 mg/mL.

In some embodiments of the present application, an aqueous solution of the pharmaceutical composition has a pH selected from 4.0-6.8; in some embodiments, an aqueous solution of the pharmaceutical composition has a pH of 4.5-6.5; in some embodiments, an aqueous solution of the pharmaceutical composition has a pH of 5.5-6.0; in some embodiments, an aqueous solution of the pharmaceutical composition has a pH of 5.5. In some embodiments, an aqueous solution of the pharmaceutical composition has a pH of 4.5, 4.8, 5.0, 5.2, 5.4, 5.5, 5.6, 5.8, or 6.0, and in some embodiments, an aqueous solution of the pharmaceutical composition has a pH of 5.0, 5.2, 5.4, 5.5, or 5.6; in some embodiments, an aqueous solution of the pharmaceutical composition has a pH of 5.5. In some embodiments, an aqueous solution of the pharmaceutical composition has a pH of 5.0. In some embodiments, an aqueous solution of the pharmaceutical composition has a pH of 5.2. In some embodiments, an aqueous solution of the pharmaceutical composition has a pH of 5.4. In some embodiments, an aqueous solution of the pharmaceutical composition has a pH of 5.5. In some embodiments, an aqueous solution of the pharmaceutical composition has a pH of 5.6. In some embodiments, an aqueous solution of the pharmaceutical composition has a pH of 5.8. In some embodiments, an aqueous solution of the pharmaceutical composition has a pH of 6.0.

In some specific embodiments of the present application, the pharmaceutical composition comprises: (a) an anti-PD-L1 antibody at a concentration of 20 mg/mL (w/v), (b) sucrose at a concentration of 70 mg/mL (w/v), (c) polysorbate 80 at a concentration of 0.1 mg/mL (w/v), (d) histidine at a concentration of 20 mM, and (e) optionally a suitable amount of hydrochloric acid for adjusting the pH of the composition to 5.0. In one specific embodiment of the present application, the pharmaceutical composition comprises: (a) an anti-PD-L1 mAb at a concentration of 20 mg/mL (w/v), (b) sucrose at a concentration of 70 mg/mL (w/v), (c) polysorbate 80 at a concentration of 0.1 mg/mL (w/v), (d) histidine at a concentration of 20 mM, and (e) optionally a suitable amount of hydrochloric acid for adjusting the pH of the composition to 5.0.

In another specific embodiment of the present application, the pharmaceutical composition comprises: (a) an anti-PD-L1 antibody at a concentration of 10 mg/mL (w/v), (b) sucrose at a concentration of 80 mg/mL (w/v), (c) polysorbate 80 at a concentration of 0.2 mg/mL (w/v), (d) histidine at a concentration of 10 mM, and (e) optionally a suitable amount of hydrochloric acid for adjusting the pH of the composition to 5.5.

In another specific embodiment of the present application, the pharmaceutical composition comprises: (a) an anti-PD-L1 antibody at a concentration of 50 mg/mL (w/v), (b) sucrose at a concentration of 80 mg/mL (w/v), (c) polysorbate 80 at a concentration of 0.3 mg/mL (w/v), (d) histidine at a concentration of 10 mM, and (e) optionally a suitable amount of hydrochloric acid for adjusting the pH of the composition to 5.5.

In another specific embodiment of the present application, the pharmaceutical composition comprises: (a) an anti-PD-L1 antibody at a concentration of 100 mg/mL (w/v), (b) sucrose at a concentration of 80 mg/mL (w/v), (c) polysorbate 80 at a concentration of 0.5 mg/mL (w/v), (d) histidine at a concentration of 10 mM, and (e) optionally a suitable amount of hydrochloric acid for adjusting the pH of the composition to 5.5.

In another specific embodiment of the present application, the pharmaceutical composition comprises: (a) an anti-PD-L1 antibody at a concentration of 30 mg/mL (w/v), (b) sucrose at a concentration of 80 mg/mL (w/v), (c) polysorbate 80 at a concentration of 0.2 mg/mL (w/v), (d) histidine at a concentration of 10 mM, and (e) optionally a suitable amount of hydrochloric acid for adjusting the pH of the composition to 5.5.

In another specific embodiment of the present application, the pharmaceutical composition comprises: (a) an anti-PD-L1 antibody at a concentration of 60 mg/mL (w/v), (b) sucrose at a concentration of 80 mg/mL (w/v), (c) polysorbate 80 at a concentration of 0.2 mg/mL (w/v), (d) histidine at a concentration of 10 mM, and (e) optionally a suitable amount of hydrochloric acid for adjusting the pH of the composition to 5.5.

In another specific embodiment of the present application, the pharmaceutical composition comprises: (a) an anti-PD-L1 antibody at a concentration of 10 mg/mL (w/v), (b) sucrose at a concentration of 70 mg/mL (w/v), (c) polysorbate 80 at a concentration of 0.4 mg/mL (w/v), (d) histidine at a concentration of 20 mM, and (e) optionally a suitable amount of acetic acid for adjusting the pH of the composition to 6.5.

In another specific embodiment of the present application, the pharmaceutical composition comprises: (a) an anti-PD-L1 mAb at a concentration of 10 mg/mL (w/v), (b) sucrose at a concentration of 80 mg/mL (w/v), (c) polysorbate 80 at a concentration of 0.2 mg/mL (w/v), (d) histidine at a concentration of 20 mM, and (e) optionally a suitable amount of hydrochloric acid for adjusting the pH of the composition to 5.5.

In another specific embodiment of the present application, the pharmaceutical composition is a water-soluble injection. In some embodiments, the water-soluble injection includes, but is not limited to, a water-soluble formulation without lyophilization or a water-soluble formulation reconstituted from a lyophilized powder. In other embodiments, the pharmaceutical composition is a lyophilized formulation. The lyophilized formulation refers to a formulation prepared by subjecting an aqueous solution to a lyophilization process in which a substance is first frozen, and then the amount of a solvent is reduced by sublimation (primary drying process) and then by desorption (secondary drying process) until the amount of the solvent is reduced to a value that no longer supports a biological activity or a chemical reaction. The lyophilized formulations of the present application can also be dried by other methods known in the art, such as spray drying and bubble drying.

### Soft tissue sarcomas

In some embodiments of the present application, the soft tissue sarcoma is advanced soft tissue sarcoma; in some embodiments, the soft tissue sarcoma is metastatic soft tissue sarcoma; in some embodiments, the soft tissue sarcoma is refractory soft tissue sarcoma; in some embodiments, the soft tissue sarcoma is advanced soft tissue sarcoma that has been subjected to chemotherapy; in some embodiments, the soft tissue sarcoma is a soft tissue sarcoma that has failed at least one chemotherapy regimen; in some embodiments, the soft tissue sarcoma is a soft tissue sarcoma that has failed at least one anthracycline-containing chemotherapy regimen; in some embodiments, the soft tissue sarcoma is a soft tissue sarcoma that has failed at least one anthracycline therapy.

In some embodiments of the present application, the soft tissue sarcoma includes, but is not limited to, malignant fibrous histiocytoma, fibrosarcoma, liposarcoma, leiomyosarcoma, rhabdomyosarcoma, synovial sarcoma, dermatofibrosarcoma protuberan, malignant peripheral nerve sheath tumor, alveolar soft-part sarcoma, clear cell sarcoma, angiosarcoma, malignant mesenchymoma, epithelioid sarcoma, and undifferentiated sarcoma (such as undifferentiated pleomorphic sarcoma).

In some embodiments of the present application, the soft tissue sarcoma includes, but is not limited to synovial sarcoma, leiomyosarcoma, alveolar soft-part sarcoma, undifferentiated pleomorphic sarcoma/malignant fibrous histiocytoma, fibrosarcoma, clear cell sarcoma, and epithelioid sarcoma.

In some embodiments of the present application, the soft tissue sarcoma is selected from the group consisting of alveolar soft-part sarcoma, undifferentiated pleomorphic sarcoma, leiomyosarcoma, epithelioid sarcoma, fibrosarcoma, and synovial sarcoma.

In some embodiments of the present application, the soft tissue sarcoma is alveolar soft-part sarcoma. In some embodiments of the present application, the soft tissue sarcoma is advanced alveolar soft-part sarcoma. In some embodiments of the present application, the soft tissue sarcoma is metastatic alveolar soft-part sarcoma. In some embodiments of the present application, the soft tissue sarcoma is alveolar soft-part sarcoma that is positive for TFE3 and/or ASPL-TFE3 fusion genes. In some embodiments of the present application, the soft tissue sarcoma is alveolar soft-part sarcoma that is positive for PAS staining. In some embodiments of the present application, the soft tissue sarcoma is alveolar soft-part sarcoma that is positive for MyoD1, and/or Vim, and/or Des, and/or NSE, and/or S-100.

In some embodiments of the present application, the patient with soft tissue sarcoma has received one or more chemotherapy regimens. In some embodiments of the present application, the soft tissue sarcoma is a soft tissue sarcoma that has failed at least one chemotherapy regimen. In some embodiments, the "failure of treatment" indicates that disease progression or intolerance occurred during treatment or within 3 months of the last treatment.

In some embodiments of the present application, the drugs used for the chemotherapy include, but are not limited to, one or more of cyclophosphamide, ifosfamide, vincristine, prednisone, prednisolone, anthracyclines (including, but not limited to, adriamycin, epirubicin, pirarubicin, amrubicin, aclarubicin, idarubicin, daunorubicin and mitoxantrone), platins (including, but not limited to, oxaliplatin, cisplatin, carboplatin, nedaplatin, dicycloplatin, miriplatin and lobaplatin), camptothecin analogs (including but not limited to camptothecin, hydroxycamptothecin, irinotecan, and topotecan), taxanes (including but not limited to paclitaxel, albumin-bound paclitaxel and docetaxel), dexamethasone, methotrexate, cytarabine, bendamustine, fludarabine, mitoxantrone, etoposide, procarbazine, gemcitabine, methylprednisolone sodium succinate, mesna, 5-fluorouracil or azacitidine, and dacarbazine.

In some embodiments of the present application, the chemotherapeutic regimen includes, but is not limited to, one or more of an ABVD regimen, an AVD regimen, a B regimen, a BA regimen, a BEACOPPesc regimen, a CDOP regimen, a CEOP regimen, a CEPP regimen, a CHOP regimen, a CIFOX regimen, a COP regimen, a COPE regimen, a CVP regimen, a DA-EPOCH regimen, a DHAP regimen, a DICE regimen, an EPOCH regimen, an ESHAP regimen, an FC regimen, an FM regimen, a GCVP regimen, a GDP regimen, a GDPE regimen, a GEMOX regimen, a GVD regimen, a HyperCVAD regimen, an ICE regimen, an IGEV regimen, an MA regimen, an MINE regimen, a miniBEAM regimen, an NCE regimen, a Stanford V regimen, and a VCAP regimen. Preferably, the chemotherapeutic regimen comprises one or more of an ABVD regimen, an AVD regimen, a BEACOPPesc regimen, a CDOP regimen, a CHOP regimen, a DA-EPOCH regimen, an EPOCH regimen, a GVD regimen, a HyperCVAD regimen, a Stanford V regimen, and a VCAP regimen.

### Regimen for therapeutic combination

In some embodiments of the present application, in the use or treatment method described above, the anti-PD-L1 antibody and anlotinib or the pharmaceutically acceptable salt thereof are each in the form of a pharmaceutical composition and can be administered simultaneously, nonsimultaneously, or sequentially.

In some embodiments of the present application, in the use or treatment method described above, the anti-PD-L1 antibody and anlotinib are separately administered at intervals. In some embodiments, the antibody and anlotinib are administered separately on the same regimen or different regimens. In some embodiments, the two are separately administered on different regimens.

In some embodiments of the present application, in the use or treatment method described above, the anti-PD-L1 antibody can be administered once every 1 week (q1w), once every 2 weeks (q2w), once every 3 weeks (q3w), or once every 4 weeks (q4w). In one specific embodiment, the anti-PD-L1 antibody is administered once every 3 weeks. In some embodiments, the anti-PD-L1 antibody is administered at a dose of 600-2400 mg each time. Anlotinib or the pharmaceutically acceptable salt thereof can be administered at a dose of 6 mg, 8 mg, 10 mg or 12 mg once daily on a regimen of consecutively 2-week treatment and then 1-week interruption.

In some embodiments, the anti-PD-L1 antibody and anlotinib or the pharmaceutically acceptable salt thereof are used in identical or different treatment cycles. In some specific embodiments, the anti-PD-L1 antibody and anlotinib are used in identical treatment cycles, e.g., in 1-week, 2-week, 3-week or 4-week treatment cycles.

In some embodiments of the present application, in the use or treatment method, one treatment cycle comprises 21 days; the PD-L1 antibody is administered on the first day of each treatment cycle, and anlotinib or the pharmaceutically acceptable salt thereof is administered daily on days 1-14 of each cycle. In one specific embodiment, the PD-L1 antibody is administered once on the first day of each treatment cycle, and anlotinib or the pharmaceutically acceptable salt thereof is administered once daily on days 1-14 of each cycle.

In some embodiments of the present application, in the use or treatment method, the anti-PD-L1 antibody can be administered to a subject at a dose selected from the group consisting of 0.01 to 40 mg/kg, 0.1 to 30 mg/kg, 0.1 to 20 mg/kg, 0.1 to 15 mg/kg, 0.1 to 10 mg/kg, 1 to 15 mg/kg, 1 to 20 mg/kg, 1 to 3 mg/kg, 3 to 10 mg/kg, 3 to 15 mg/kg, 3 to 20 mg/kg, 3 to 30 mg/kg, 10 to 20 mg/kg, or 15 to 20 mg/kg, or administered to a subject at a dose of 60 mg to 2400 mg, 90 mg to 1800 mg, 120 mg to 1500 mg, 300 mg to 900 mg, 600 mg to 900 mg, 300 mg to 1200 mg, 600 mg to 1200 mg, or 900 mg to 1200 mg.

In some embodiments for the use or treatment method, 21 days are counted as one treatment cycle, 1200 mg of the PD-L1 antibody is administered on the first day of each treatment cycle, and 6 mg, 8 mg, 10 mg and/or 12 mg of anlotinib or the pharmaceutically acceptable salt thereof is administered daily on days 1-14 of each cycle.

### Administration

The content below is not intended to limit the administration of the therapeutic combination disclosed herein.

The components in the therapeutic combination disclosed herein can be administered independently, or some or all of the components are co-administered in various proper routes, including, but not limited to, oral administration or parenteral administration (by intravenous, intramuscular, local or subcutaneous routes). In some embodiments, the components in the therapeutic combination disclosed herein can be administered independently, or some or all of the components are co-administered by means of oral administration or injection, for example, intravenous injection or intraperitoneal injection.

The components of the therapeutic combination of the present application can be independent suitable dosage forms, or some or all of the components are co-formulated in a suitable dosage form including but not limited to, tablet, lozenge, pill, capsule (for example, hard capsule, soft capsule, enteric capsule and microcapsule), elixir, granule, syrup, injection (intramuscular, intravenous and intraperitoneal), granule, emulsion, suspension, solution, dispersant and dosage forms of sustained-release formulations for oral or non-oral administration.

The components in the therapeutic combination disclosed herein can be formulated independently, or some or all of the components are co-formulated with a pharmaceutically acceptable carrier and/or excipient.

In some embodiments, the therapeutic combination of the present application can safely and effectively treat advanced soft tissue sarcoma, particularly advanced soft tissue sarcomas that have been treated with a chemotherapeutic regimen, and more particularly advanced soft tissue sarcomas that have been treated with an anthracycline. In some embodiments, the treatment is very effective for alveolar soft-part sarcoma.

In some embodiments, the ORR of a soft tissue sarcoma patient is up to or greater than 50% after receiving the drug combination therapy of the present application. In some embodiments, the ORR is up to or greater than 60%. In some embodiments, the ORR is up to or greater than 70%. In some embodiments, the therapeutic combination of the present application exhibits superior anti-tumor effect in the treatment of alveolar soft-part sarcoma, and the ORR of the patient is up to or greater than 63.6% after receiving the treatment. In some embodiments, the ORR is up to or greater than 75%.

The therapeutic combination or the pharmaceutical composition of the present application can be effective in treating soft tissue sarcoma, with advantages including, but not limited to, one or more of the following:
(1) better efficacy in controlling tumor growth or even eliminating tumors as compared with either drug of the combination administered alone;
(2) fewer doses as compared with either drug of the combination administered alone;
(3) good tolerability in patients, and/or fewer adverse effects or complications as compared with either drug administered alone;
(4) a higher disease control rate in patients treated;
(5) longer survivals (e.g., median survival, progression-free survival, or overall survival) in patients treated;
(6) longer survivals (e.g., median survival, progression-free survival, or overall survival) in patients treated as compared with standard chemotherapies;
(7) a longer duration of response (DOR); and/or
(8) better activity in treating soft tissue sarcoma and better anti-tumor synergistic effect, as compared with either drug of the combination administered alone.

### Definitions and Description

Unless otherwise stated, the following terms used in the present application shall have the following meanings. A certain term, unless otherwise specifically defined, should not be considered uncertain or unclear, but construed according to its common meaning in the field. When referring to a trade name, it is intended to refer to its corresponding commercial product or its active ingredient.

Herein, unless otherwise specified, the amount of anlotinib or a pharmaceutically acceptable salt thereof mentioned herein refers to the amount of the active ingredient anlotinib free base.

Unless otherwise specified, the term "dose" refers to a dose administered to a patient without considering the weight or the body surface area (BSA) of the patient. For example, a 60 kg human and a 100 kg human will receive the same dose of antibody (e.g., 240 mg of anti-PD-1 antibody).

As used herein, for drugs used in the chemotherapeutic regimen, reference may be made to the following, or treatment guidelines or textbooks relating to medicine and pharmacy:
ABVD: doxorubicin, bleomycin, vinblastine and dacarbazine.
AVD: doxorubicin, vinblastine and dacarbazine.
B: bendamustine.
BEACOPPesc regimen: etoposide, doxorubicin, cyclophosphamide, vincristine, bleomycin, prednisone and procarbazine.
CDOP: cyclophosphamide, doxorubicin, vincristine and prednisone.
CEOP: cyclophosphamide, etoposide, vincristine and prednisone.
CEPP cyclophosphamide, etoposide, prednisolone and procarbazine.
CHOP: cyclophosphamide, doxorubicin, vincristine and prednisone.
CIFOX: 5-fluorouracil and oxaliplatin.
COP: cyclophosphamide, vincristine and prednisone.
COPE: cyclophosphamide, vincristine, cisplatin and etoposide.
CVP: cyclophosphamide, vincristine and prednisone.
DA-EPOCH: etoposide, prednisone, vincristine, cyclophosphamide and adriamycin.
DHAP: dexamethasone, high-dose cytarabine and cisplatin.
DICE: dexamethasone, ifosfamide, cisplatin and etoposide.
EPOCH: etoposide, prednisone, vincristine, cyclophosphamide and adriamycin.
ESHAP: etoposide, methylprednisolone sodium succinate, high-dose cytarabine and cisplatin.
FC: fludarabine and cyclophosphamide.
FM: fludarabine and mitoxantrone.
GCVP: gemcitabine, cyclophosphamide, vincristine and prednisone.
GDP: gemcitabine, dexamethasone and cisplatin.
GDPE: gemcitabine, dexamethasone, cisplatin and etoposide.
GEMOX: gemcitabine and oxaliplatin.
GVD: gemcitabine, vinorelbine and doxorubicin.
HyperCVAD: cyclophosphamide, vincristine, adriamycin and dexamethasone.
ICE: ifosfamide, cisplatin and etoposide.
IGEV regimen: ifosfamide, gemcitabine and vinorelbine.
MA: methotrexate and cytarabine.
MINE: mesna, ifosfamide, mitoxantrone and etoposide.
miniBEAM: carmustine, etoposide, cytarabine and melphalan.
NCE: vinorelbine, cisplatin and etoposide.

Stanford V regimen: doxorubicin, vinblastine, nitrogen mustard, vincristine, bleomycin, etoposide and prednisone. V-CAP: bortezomib, cyclophosphamide, adriamycin and prednisone.

Herein, "complete response" (CR) refers to disappearance of all target lesions.

Herein, "partial response" (PR) refers to a reduction in the total diameter of the target lesion by 30% or more compared to the baseline total diameter.

Herein, "disease progression" (PD) refers to an increase in the total diameter of the target lesion by 20% or more compared to the minimum value of the total diameter found in the study, including the baseline total diameter if it is the minimum value in the study. Besides the relative increase of 20 % or more, the absolute value of the total diameter must be increased by 5 mm or more. The appearance of one or more new lesions should also be considered as disease progression.

Herein, "stable disease" (SD) refers to the shrinkage of the lesion, but not enough for the PR standard; or the enlargement of the lesion, but not enough for the PD standard, and the minimum diameter was used as a reference in the test.

Herein, "best efficacy" refers to the best therapeutic effect at all time points from initial assessment, by the end of the last assessment.

Herein, "TFE 3" refers to Transcription Factor E3, also known as Transcription Factor Binding To IGHM Enhancer 3.

Herein, "ASPL" refers to the alveolar soft-part sarcoma gene.

Herein, "positive for PAS staining" means that the cytoplasm of tumor cells contains glycogen and a large number of rod-shaped or rod-shaped purple-red crystals that are resistant to amylase digestion, as shown by PAS staining (Periodic Acid-Schiff stain).

Herein, "MyoD1" refers to myogenic transcriptional regulatory protein (Myogenic Differentiation 1).

Herein, "Vim" refers to Vimentin.

Herein, "Des" refers to Desmin.

Herein, "NSE" refers to neuron-specific enolase.

Herein, "S-100" refers to a water-soluble protein with a molecular weight of 21 kDa, which is isolated from the central nervous system and named because it is 100% soluble in an ammonium sulfate solution.

As used herein, the term "therapeutic combination" refers to a combination of two or more active ingredients (administered as the respective active ingredients themselves, or as their respective derivatives like pharmaceutically acceptable salts or esters, prodrugs, or compositions) that are administered simultaneously or sequentially. The active substances can be administered to a subject simultaneously or sequentially in any order as a single formulation.

As used herein, the term "antibody" refers to a binding protein having at least one antigen-binding domain. The antibody and the fragment thereof of the present application can be an intact antibody or any fragment thereof. Thus, the antibody and the fragment thereof of the present application include a monoclonal antibody or a fragment thereof and an antibody variant or a fragment thereof, as well as an immunoconjugate. Examples of the antibody fragment include an Fab fragment, an Fab' fragment, an F(ab')₂ fragment, an Fv fragment, an isolated CDR region, a single chain Fv molecule (scFv), an Fd fragment and other antibody fragments known in the art. The antibody and the fragment thereof may also include a recombinant polypeptide, a fusion protein, and a bispecific antibody. The anti-PD-L1 antibody and the fragment thereof described herein can be of IgG1, IgG2, IgG3, or IgG4 isotype. The term "isotype" refers to the class of antibodies encoded by the heavy chain constant region gene. In one embodiment, the anti-PD-L1 antibody and the fragment thereof described herein are of the IgG1 or IgG4 isotype. The anti-PD-L1 antibody and the fragment thereof of the present application can be derived from any species including, but not limited to, mouse, rat, rabbit, primate, llama, and human. The anti-PD-L1 antibody and the fragment thereof can be a chimeric antibody, a humanized antibody or an intact human antibody. In one embodiment, the anti-PD-L1 antibody is an antibody produced by a hybridoma cell line derived from a mouse. Thus, in one embodiment, the anti-PD-L1 antibody is a murine antibody. In another embodiment, the anti-PD-L1 antibody is a chimeric antibody. In another embodiment, the chimeric antibody is a mouse-human chimeric antibody. In another embodiment, the antibody is a humanized antibody. In another embodiment, the antibody is derived from a murine antibody and is humanized.

The "humanized antibody" refers to an antibody comprising complementarity determining regions (CDRs) derived from a non-human antibody, and framework and constant regions derived from a human antibody. For example, an anti-PD-L1 antibody described herein may comprise CDRs derived from one or more murine antibodies as well as human framework and constant regions. Thus, in one embodiment, the humanized antibody described herein binds to the same epitope on PD-L1 as the murine antibody from which the CDRs of the humanized antibody are derived. In some embodiments, the anti-PD-L1 antibody is a humanized antibody. Additional anti-PD-L1 antibodies or variants thereof comprising the heavy and light chain CDRs disclosed herein can be generated using any human framework sequences, and are also included in the present application. In one embodiment, framework sequences suitable for use in the present application include those similar in structure to the framework sequences disclosed herein. Additional modifications may be made in the framework regions to improve the properties of the antibodies provided herein. Such additional framework modifications may include: chemical modifications, point mutations for reducing immunogenicity or removing T cell epitopes, or modifications reverting the mutations to residues in original germline sequences. In some embodiments, such modifications include those corresponding to the mutations exemplified herein, including reversions to germline sequences. For example, in one embodiment, one or more amino acids in the human VH and/or VL framework regions of the humanized antibodies described herein are reverted to the corresponding amino acids in the parent murine antibodies. For example, for the VH and VL of humanized 5G11 and humanized 13C5 antibodies, several sites of framework amino acids of the template human antibodies described above are reverted to the corresponding amino acid sequences in the mouse 5G11 and 13C5 antibodies. In one embodiment, the amino acids at positions 53, 60 and/or 67 of the light chain variable region are reverted to the corresponding amino acids found at the positions in mouse 5G11 or 13C5 light chain variable region. In another embodiment, the amino acids at positions 24, 28, 30, 49, 73, 83 and/or 94 of the heavy chain variable region are reverted to the corresponding amino acids found at the positions in mouse 5G11 or 13C5 heavy chain variable region. In one embodiment, the humanized 5G11 antibody comprises: a light chain variable region, wherein the amino acid at position 60 is mutated from Ser (S) to Asp (D) and the amino acid at position 67 is mutated from Ser (S) to Tyr (Y); and a heavy chain variable region, wherein the amino acid at position 24 is mutated from Phe (F) to Val (V), the amino acid at position 49 is mutated from Ala (A) to Gly (G), the amino acid at position 73 is mutated from Thr (T) to Asn (N), and the amino acid at position 83 is mutated from Thr (T) to Asn (N). In one embodiment, the humanized 13C5 antibody comprises: a light chain variable region, wherein the amino acid at position 53 is mutated from Tyr (Y) to Lys (K); and a heavy chain variable region, wherein the amino acid at position 28 is mutated from Thr (T) to Ile (I), the amino acid at position 30 is mutated from Ser (S) to Arg (R), the amino acid at position 49 is mutated from Ser (S) to Ala (A), and the amino acid at position 94 is mutated from Tyr (Y) to Asp (D). Additional or alternative reverse mutations can be made in the framework regions of the humanized antibodies provided herein to improve the properties of the antibodies. The present application also includes humanized antibodies that bind to PD-L1 and comprise framework modifications corresponding to the exemplary modifications disclosed herein relative to any suitable framework sequence, as well as other framework modifications that otherwise improve antibody properties.

The present application provides an isolated antibody or fragment thereof that binds to PD-L1, wherein the antibody can be produced by a hybridoma selected from the group consisting of the hybridomas designated herein as 13C5 and 5G11. Accordingly, the antibody of the present application further includes hybridomas 13C5 and 5G11, and any hybridomas that produce the antibodies disclosed herein. The present application further comprises isolated polynucleotides encoding the antibodies and the fragments thereof disclosed herein. The present application also includes expression vectors comprising the isolated polynucleotides, and host cells comprising the expression vectors.

The "isolated antibody" refers to an antibody that is substantially free of other antibodies having different antigenic specificities (e.g., an isolated antibody that specifically binds to PD-1 is substantially free of antibodies that specifically bind to antigens apart from PD-1). However, an isolated antibody that specifically binds to PD-1 may have cross-reactivity with other antigens (such as PD-1 molecules from different species). Furthermore, the isolated antibody may be substantially free of other cellular materials and/or chemicals.

The term "monoclonal antibody" ("mAb") refers to a non-naturally occurring preparation of antibody molecules of an individual molecule component (i.e., antibody molecules whose base sequences are substantially identical and which exhibit a single binding specificity and affinity for a particular epitope). mAb is an example of the isolated antibody. mAbs can be produced by hybridoma techniques, recombinant techniques, transgenic techniques, or other techniques known to those skilled in the art.

The antibody or the antigen-binding fragment thereof described herein is specific for PD-L1. In one embodiment, the antibody or the fragment thereof is specific for PD-L1. In one embodiment, the antibody or the fragment thereof described herein binds to human or primate PD-L1, but does not bind to PD- L1 from any other mammals. In another embodiment, the antibody or the fragment thereof does not bind to mouse PD-L1. The terms "human PD-L1", "hPD-L1" and "huPD-L1" and the like are used interchangeably herein and refer to human PD-L1 and variants or isotypes of human PD-L1. The "specific", "specificity" and "specifically" refer to that the antibody or fragment thereof binds to PD-L1 with greater affinity than any other targets.

The terms "treat", "treating" and "treatment" usually refer to acquiring needed pharmacological effect and/or physiological effect. In terms of partially or fully stabilizing or curing the disease and/or a side effect of the disease, the effect can be therapeutic. As used herein, "treat" and "treatment" encompass any treatment to a disease in a patient, including (a) inhibiting a symptom of a disease, i.e., blocking the progression of the disease; or (b) alleviating a symptom of a disease, i.e., causing remission of the disease or the symptom.

The term "effective amount" refers to an amount of the compound disclosed herein for (i) treating a specific disease, condition or disorder; (ii) alleviating, improving or eliminating one or more symptoms of a specific disease, condition or disorder, or (iii) preventing or delaying onset of one or more symptoms of a specific disease, condition or disorder described herein. The amount of active substance (e.g., the antibody or compound disclosed herein) constituting a "therapeutically effective amount" may vary according to factors such as the disease state, age, sex, and weight of the individual, and the ability of a therapeutic agent or a combination of therapeutic agents to elicit a desired response in the individual. The effective amount may also be determined routinely by those skilled in the art in accordance with their knowledge and the present disclosure.

The terms "administer", "administration" and "administering" refer to physically introducing the composition comprising a therapeutic agent to an entity using any of a variety of methods and delivery systems known to those skilled in the art. Routes of administration of immune checkpoint inhibitors (e.g., an anti-PD-1 antibody or an anti-PD-L1 antibody) include parenteral routes of administration (including but not limited to intravenous, intramuscular, subcutaneous, intraperitoneal, spinal, or other parenteral routes of administration), for example, by injection or infusion. In some embodiments, the phrase "parenteral administration" or "administered parenterally" as used herein are used interchangeably, and typically refer to modes of administration apart from enteral and local administration, typically by injection, including but not limited to, intravenous, intramuscular, intra-arterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion and in vivo electroporation. In some embodiments, the immune checkpoint inhibitor (e.g., an anti-PD-1 antibody or an anti-PD-L1 antibody) is administered by a non-parenteral route, and in some embodiments, by oral administration; other non-parenteral routes include local, epidermal or mucosal administration route, e.g., intranasal, intravaginal, intrarectal, sublingual or local administration route. Administration may also be performed, e.g., once, multiple times, and/or over one or more extended periods of time.

The term "pharmaceutically acceptable" is used herein for those compounds, materials, compositions, and/or dosage forms which are suitable for use in contact with the tissues of human beings and animals without excessive toxicity, irritation, allergic response, or other problems or complications.

The term "pharmaceutically acceptable salt" includes salts formed from a free base and an acid and salts formed from an acid and a free base, for example, hydrochloride, hydrobromide, nitrate, sulfate, phosphate, formate, acetate, trifluoroacetate, fumarate, oxalate, maleate, citrate, succinate, mesylate, benzenesulfonate and p-methylbenzenesulfonate; in some embodiments, the salts are hydrochloride, hydrobromide, sulfate, formate, acetate, trifluoroacetate, fumarate, maleate, mesylate, p-methylbenzenesulfonate, sodium salt, potassium salt, ammonium salt and amino acid salt, and the like. In the present application, in forming a pharmaceutically acceptable salt, the acid and the free base are in a molar ratio of 1:0.2-1:5; in some embodiment, the acid and the free base are in a molar ratio of 1:0.5, 1:1, 1:2, 1:3, 1:4, 1:5, 1:6, 1:7, or 1:8.

Herein, the terms "individual", "subject" and "patient" are used interchangeably herein. Herein, the "patient" is a mammal, and in some embodiments, the patient is a human. In some embodiments, the subject is a human.

The term "unit dose" refers to the smallest unit of packaging containing a certain quantity of pharmaceutical product; for example, in a box of seven capsules, each capsule is a unit dose; or a vial of injection is a unit dose. As used herein, the terms "single dose" and "unit dose" have the same meaning and are used interchangeably.

The term "multiple dose" consists of multiple unit doses.

The term "pharmaceutical composition" refers to a mixture consisting of one or more of the active ingredients or therapeutic combinations thereof of the present application and a pharmaceutically acceptable excipient. The pharmaceutical composition is intended to facilitate the administration of the compound or the therapeutic combination thereof disclosed herein to a patient.

As used herein, unless otherwise stated, the terms "comprise", "comprises" and "comprising" or equivalents thereof are open-ended statements and mean that elements, components and steps that are not specified may be included in addition to those listed.

As used herein, unless otherwise indicated, all numbers expressing the amounts of ingredients, measurements, or reaction conditions used herein are to be understood as being modified in all instances by the term "about". The term "about" when connected to a percentage may mean, for example, ± 0.1%, preferably, ± 0.05%, and more preferably, ± 0.01%.

Unless otherwise specified clearly herein, singular terms encompass plural referents, and vice versa. Similarly, unless otherwise specified clearly herein, the word "or" is intended to include "and".

All patents, patent applications and other publications are explicitly incorporated herein by reference for the purpose of description and disclosure. These publications are provided solely because they were disclosed prior to the filing date of the present application. All statements as to the dates of these documents or description as to the contents of these documents are based on the information available to the applicant and do not constitute any admission as to the correctness of the dates or the content of these documents. Moreover, in any country or region, any reference to these publications herein is not to be construed as an admission that the publications form part of the commonly recognized knowledge in the art.

### DETAILED DESCRIPTION

For clarity, the present application is further described with the following examples, which are, however, not intended to limit the scope of the present application. All reagents are commercially available and can be used without further purification. In the examples, the anti-PD-L1 antibody may be prepared as described in WO2016022630, and after affinity chromatography, an eluate containing the antibody was obtained by conventional antibody purification methods.

### Example

### Example 1. Clinical Trial of Soft Tissue Sarcoma

The anti-PD-L1 antibody injection and the Anlotinib hydrochloride capsule were administered to soft tissue sarcoma subjects meeting the inclusion criteria, for patients with disease under control (CR + PR + SD) and tolerable adverse reactions, the administration could be continued, and the study ended when the investigator considered that it was not suitable to continue the administration, for example, disease progression occurred, clinical benefits were lost, the toxicity was intolerable and cannot be relieved, or the subjects decided to withdraw. The main research objective of this clinical trial was to evaluate the safety and initial efficacy of adminitration.

### 1.1 Primary inclusion criteria:

1) Pathologically confirmed advanced soft tissue sarcoma with at least one measurable lesion, and the pathological subtypes of soft tissue sarcoma including, but not limited to synovial sarcoma, leiomyosarcoma, alveolar soft-part sarcoma, undifferentiated pleomorphic sarcoma/malignant fibrous histiocytoma, fibrosarcoma, clear cell sarcoma, and epithelioid sarcoma;
2) Patients who failed at least 1 chemotherapeutic regimen (necessarily anthracycline-containing) of other types of soft tissue sarcomas except for alveolar soft-part sarcoma and clear cell sarcoma and who progressed or were intolerant for 6 months prior to enrollment;
3) Age: 18-70 years; ECOG PS scoring: 0-1; an expected survival time of more than 3 months.

### 1.2 Test drug

Anti-PD-L1 antibody injection hu5G 11-hIgG 1: 1200 mg of the anti-PD-L1 antibody injection (strength: 100 mg/10 mL) was diluted to 250 mL with normal saline, and the infusion time was 60 ± 5 min (from the beginning of the infusion of the anti-PD-L1 antibody injection until the end of the infusion of the anti-PD-L1 antibody injection and the completion of flushing the tube with normal saline (20 mL recommended)). The anti-PD-L1 antibody injection was administered on the first day, and once every 21 days, that is, 21 days were counted as a treatment cycle (d1/q3w). The longest administration time was no more than 24 months.

Anlotinib hydrochloride capsule (anlotinib dihydrochloride as active ingredient): once daily (administrated orally at fasting within 5 minutes before or after the start of anti-PD-L1 antibody infusion), 1 capsule (12 mg) each time. The oral administration was performed on a regimen of consecutively 2-week oral administration and then 1-week interruption, that is, 21 days were counted as a treatment cycle, and the drug was administered on days 1-14 of each cycle. Except in special circumstances, it was recommended to take it at a fixed time every day. (i.e., anlotinib hydrochloride capsule: 12 mg/qd, d1-14/q3w)
The investigator could adjust the dose of the anlotinib hydrochloride capsule, e.g., to 12 mg, 10 mg or 8 mg, according to the disease condition, safety and other aspects.

### 1.3 Evaluation criteria

Evaluation criteria for safety: NCI-CTC AE 5.0 criteria for the adverse events of the test drug.

Effectiveness evaluation criteria: disease status was determined using RECIST 1.1, iRECIST criteria. RECIST 1.1 criteria were used as the primary criteria, and iRECIST criteria were used to confirm efficacy. That is, subjects determined to be disease progression (PD) according to RECIST 1.1 criteria were further confirmed according to iRECIST criteria to determine whether to take further medication observation.

### 1.4 Research endpoints

Primary endpoint: objective response rate (ORR = CR + PR)
Secondary endpoint: (1) an adverse event; drug dose adjustment; (2) progression-free survival (PFS), disease control rate (DCR = CR + PR + SD), overall survival (OS), and the like; (3) detection of tumor markers such as PD-L1 expression, TMB, MSI, and the like.

### 1.5 Results

The prior research result showed that the combined therapeutic combination could safely and effectively treat the soft tissue sarcoma. In 29 soft tissue sarcoma subjects, the best curative effect of 9 subjects reached PR, the best curative effect of 14 subjects reached SD, and DCR reached 79.3%. The evaluation of the curative effect of undifferentiated pleomorphic sarcoma and leiomyosarcoma showed that the DCR reached 80% and 75% respectively; after 6 cycles of treatment, the tumor of the subjects with epithelioid sarcoma contracted by 60% and the disease was relieved. After 4 cycles of treatment, the tumor size of subject with synovial sarcoma remained essentially stable and the disease was controlled.

In a prior clinical study, the ORR was greater than 63.6%, the DCR was greater than 90.9%, and the median PFS of the subjects reached or exceeded 11.7 months in 11 subjects with evaluable alveolar soft-part sarcoma.

As the latest clinical research data showed, the subjects with the evaluable alveolar soft-part sarcoma reached 28 cases, and the research results showed that the drug combination had significant curative effect on the alveolar soft-part sarcoma, the ORR reached or exceeded 75%, and the DCR reached 100%. In addition, it was expected that as the treatment cycle was extended, the median PFS of the subject would be further extended. The efficacy data for a part of the subjects with alveolar soft-part sarcoma are shown in table 1.

**Table 1 Efficacy data for a part of subjects with alveolar soft-part sarcoma**

| Subjects No. | Best efficacy | Enrolled Time | C0 | C2 | C4 | C6 | C8 | C10 | C12 | C14 | C16 | C18 | C20 |
|---|---|---|---|---|---|---|---|---|---|---|---|---|---|
| 1001 | PR | 2019.03 | 74 | 52 | 42 | 42 | 38 | 30 | 22 | / | / | / | 16 (C21) |
| 1012 | PR | 2019.05 | 39 | 35 | 32 | 28 | 22 | 19 | / | 14 (C15) | 14 (C17) | 14 | 14 |
| 1014 | PR | 2019.05 | 165 | 113 | 97 | 86 | 76 | 76 | / | 63 (C15) | 51 (C17) | 61 | 61 |
| 1017 | PR | 2019.06 | 61 | 72 | 61 | 51 | 49 | / | / | 44 (C15) | 40 (C17) | 39 | 37 |
| 1022 | PR | 2019.07 | 74 | 46 | 38 | 31 | 31 | / | 25 (C13) | 23 (C15) | 25 | 16 | 15.3 |
| 1024 | SD | 2019.08 | 94 | 96 | 106 | 110 | / | 110 (C11) | 122 | 113 | 113 | 117 | 123 |
| 1025 | CR | 2019.08 | 14 | 11 | 8 | 8 | / | / | 5 (C13) | 6 | 5 | 0 | 0 |
| 1027 | SD | 2019.10 | 32 | 30 | 30 | / | / | / | 40 (C11) | 42 | 44 | / | / |
| 1028 | PR | 2019.10 | 19 | 17 | 16 | / | / | 11 (C11) | 11 | 11 | 11 | 11 | 11 |
| 2004 | SD | 2019.07 | 55.1 | 53.4 | 55.3 | 53 | 52.7 | / | / | / | 58.8 | 58.6 | 60.7 |

In the table, "Cn" (e.g., n = 0, 2, 4, 6, 8, 10, 12, 14, 16, 18, 20, 21) means the longest diameter or the sum of the longest diameters, in millimeters, of the target lesion in the subject is recorded after measuring the target lesion by imaging examination or clinical examination according to the evaluation criteria described in item 1.3 after n treatment cycles (one treatment cycle every 3 weeks) of the combined therapeutic combination; when the table has treatment periods indicated in parentheses, the treatment periods in the parentheses are taken as the basis; "/" means that the subject is not scheduled to the hospital for evaluation due to due to personal or external reasons (e.g., novel coronavirus); or the evaluation time is not reached. Recent clinical studies have demonstrated that the therapeutic combination has significant therapeutic effects on soft tissue sarcoma, with ORR being over 50% and DCR being over 90%, and the statistical data of the efficacy of some subjects are shown in table 2.

**Table 2**

| | Number of subjects (ratio) | | | | |
|---|---|---|---|---|---|
| efficacy | Alveolar soft-part sarcoma | Leiomyosarcoma | Epithelioid sarcoma | Undifferentiated pleomorphic sarcoma/malignant fibrous histiocytoma | Fibrosarcoma |
| CR | 1/28 (3.57%) | 0/4 (0.00%) | 0/1 (0.00%) | 0/2 (0.00%) | 0/2 (0.00%) |
| PR | 20/28 (71.43%) | 1/4 (25.00%) | 1/1 (100.00%) | 0/2 (0.00%) | 0/2 (0.00%) |
| SD | 7/28 (25.00%) | 3/4 (75.00%) | 0/1 (0.00%) | 2/2 (100.00%) | 2/2 (100.00%) |
| PD | 0/28 (0.00%) | 0/4 (0.00%) | 0/1 (0.00%) | 0/2 (0.00%) | 0/2 (0.00%) |
| ORR | 21/28 (75.00%) | 1/4 (25.00%) | 1/1 (100.00%) | 0/2 (0.00%) | 0/2 (0.00%) |
| DCR | 28/28 (100.00%) | 4/4 (100.00%) | 1/1 (100.00%) | 2/2 (100.00%) | 2/2 (100.00%) |

Among them, epithelioid sarcoma has 1 evaluable case, and the disease is relieved after receiving treatment, and the tumor almost disappears, and it is expected that complete response can be achieved with the extension of treatment period. Undifferentiated pleomorphic sarcoma/malignant fibrous histiocytoma and fibrosarcoma each have 2 evaluable cases, and the diseases all are controlled after treatment. Leiomyosarcoma has 4 evaluable cases, and the disease in 1 case is relieved and diseases in 3 cases are controlled after treatment. In addition, the best efficacy achieved SD in 4 of 7 synovial sarcoma subjects.

Those skilled in the art will recognize that the scope of the present application is not limited to the various embodiments and examples described above. Instead, various modifications, substitutions, or recombinations can be made without departing from the spirit of the present application, all of which fall within the protection scope of the present application.

## Claims

1. Use of a therapeutic combination comprising an anti-PD-L1 antibody, and anlotinib or a pharmaceutically acceptable salt thereof in preparing a medicament for treating soft tissue sarcoma, wherein the anti-PD-L1 antibody comprises the following amino acid sequences: a heavy chain CDR1 region having at least 80% homology to an amino acid sequence set forth in SEQ ID NO: 1 or SEQ ID NO: 4; or a heavy chain CDR2 region having at least 80% homology to an amino acid sequence set forth in SEQ ID NO: 2 or SEQ ID NO: 5; or a heavy chain CDR3 region having at least 80% homology to an amino acid sequence set forth in SEQ ID NO: 3 or SEQ ID NO: 6; or a light chain CDR1 region having at least 80% homology to an amino acid sequence set forth in SEQ ID NO: 7 or SEQ ID NO: 10; or a light chain CDR2 region having at least 80% homology to an amino acid sequence set forth in SEQ ID NO: 8 or SEQ ID NO: 11; or a light chain CDR3 region having at least 80% homology to an amino acid sequence set forth in SEQ ID NO: 9 or SEQ ID NO: 12.

2. The use according to claim 1, wherein the anti-PD-L1 antibody, and anlotinib or the pharmaceutically acceptable salt thereof are separately packaged or packaged together.

3. The use according to claim 1 or 2, wherein the anti-PD-L1 antibody is in a form for parenteral administration or in a form for intravenous administration.

4. The use according to any one of claims 1-3, wherein the anti-PD-L1 antibody, and anlotinib or the pharmaceutically acceptable salt thereof are in a weight ratio of (0.35-29):1, (3.5-29):1, (3.5-14.5):1 or (7-14.5):1.

5. The use according to any one of claims 1-4, wherein the anti-PD-L1 antibody is prepared as a pharmaceutical composition, and the anti-PD-L1 antibody in the pharmaceutical composition is at a concentration of 10-60 mg/mL, or 10 mg/mL, 20 mg/mL, 30 mg/mL, 40 mg/mL, 50 mg/mL, or 60 mg/mL.

6. The use according to any one of claims 1-5, wherein the anti-PD-L1 antibody is prepared as a pharmaceutical composition, and the anti-PD-L1 antibody in the pharmaceutical composition is at a dose of 600-2400 mg, or 600 mg, 800 mg, 1000 mg, 1200 mg, 1400 mg, 1600 mg, 1800 mg, 2000 mg, 2200 mg, or 2400 mg.

7. The use according to any one of claims 1-6, wherein anlotinib or the pharmaceutically acceptable salt thereof is prepared as a pharmaceutical composition, wherein anlotinib or the pharmaceutically acceptable salt thereof in the pharmaceutical composition is at a dose of 6 mg to 12 mg, or 6 mg, 8 mg, 10 mg, or 12 mg.

8. The use according to any one of claims 1-7, wherein the therapeutic combination is a formulation suitable for administration within a single treatment cycle (e.g., a 21-day treatment cycle), and comprises a pharmaceutical composition comprising 600-2400 mg of the anti-PD-L1 antibody and a pharmaceutical composition comprising 84-168 mg of anlotinib or the pharmaceutically acceptable salt thereof.

9. The use according to any one of claims 1-8, wherein the soft tissue sarcoma is advanced soft tissue sarcoma.

10. The use according to any one of claims 1-9, wherein the soft tissue sarcoma is selected from the group consisting of synovial sarcoma, leiomyosarcoma, alveolar soft-part sarcoma, undifferentiated pleomorphic sarcoma/malignant fibrous histiocytoma, fibrosarcoma, clear cell sarcoma, and epithelioid sarcoma.

11. The use according to any one of claims 1-10, wherein the soft tissue sarcoma is a soft tissue sarcoma that has received and failed at least one chemotherapy regimen.

12. The use according to any one of claims 1-11, wherein the anti-PD-L1 antibody, and anlotinib or the pharmaceutically acceptable salt thereof are each in the form of a pharmaceutical composition.

13. The use according to claim 12, wherein the pharmaceutical composition of the anti-PD-L1 antibody is a solution for injection; the pharmaceutical composition of anlotinib or the pharmaceutically acceptable salt thereof is an oral solid formulation.

14. A method for treating soft tissue sarcoma, comprising: administering to a patient in need thereof a therapeutically effective amount of an anti-PD-L1 antibody, and anlotinib or a pharmaceutically acceptable salt thereof, wherein the anti-PD-L1 antibody comprises the following amino acid sequences: a heavy chain CDR1 region having at least 80% homology to an amino acid sequence set forth in SEQ ID NO: 1 or SEQ ID NO: 4; or a heavy chain CDR2 region having at least 80% homology to an amino acid sequence set forth in SEQ ID NO: 2 or SEQ ID NO: 5; or a heavy chain CDR3 region having at least 80% homology to an amino acid sequence set forth in SEQ ID NO: 3 or SEQ ID NO: 6; or a light chain CDR1 region having at least 80% homology to an amino acid sequence set forth in SEQ ID NO: 7 or SEQ ID NO: 10; or a light chain CDR2 region having at least 80% homology to an amino acid sequence set forth in SEQ ID NO: 8 or SEQ ID NO: 11; or a light chain CDR3 region having at least 80% homology to an amino acid sequence set forth in SEQ ID NO: 9 or SEQ ID NO: 12.

15. The method according to claim 14, wherein the anti-PD-L1 antibody, and anlotinib or the pharmaceutically acceptable salt thereof are each in the form of a pharmaceutical composition.

16. The method according to claim 14 or 15, wherein the anti-PD-L1 antibody, and anlotinib or a pharmaceutically acceptable salt thereof are administered simultaneously, nonsimultaneously, or sequentially.

17. The method according to any one of claims 14-16, wherein the anti-PD-L1 antibody is administered once every 1 week, every 2 weeks, every 3 weeks, or every 4 weeks.

18. The method according to any one of claims 14-17, wherein the anti-PD-L1 antibody is administered at a dose of 600-2400 mg each time.

19. The method according to any one of claims 14-18, wherein the anti-PD-L1 antibody is administered at a single dose of 600 mg, 800 mg, 1000 mg, 1200 mg, 1400 mg, 1600 mg, 1800 mg, 2000 mg, 2200 mg, or 2400 mg.

20. The method according to any one of claims 14-19, wherein anlotinib or the pharmaceutically acceptable salt thereof is administered at a daily dose of 6 mg to 12 mg.

21. The method according to any one of claims 14-20, wherein every 3 weeks are counted as one treatment cycle, the anti-PD-L1 antibody is administered on the first day of each cycle, and anlotinib or the pharmaceutically acceptable salt thereof is administered on days 1-14 of each cycle.

22. The method according to any one of claims 14-21, wherein the anti-PD-L1 antibody is administered at a dose of 600-2400 mg per treatment cycle, and a total dose of anlotinib or the pharmaceutically acceptable salt thereof administered per treatment cycle is 84-168 mg.

23. The method according to any one of claims 14-22, wherein the soft tissue sarcoma is advanced soft tissue sarcoma.

24. The method according to any one of claims 14-23, wherein the soft tissue sarcoma is selected from the group consisting of synovial sarcoma, leiomyosarcoma, alveolar soft-part sarcoma, undifferentiated pleomorphic sarcoma/malignant fibrous histiocytoma, fibrosarcoma, clear cell sarcoma, and epithelioid sarcoma.

25. The method according to any one of claims 14-24, wherein the soft tissue sarcoma is a soft tissue sarcoma that has received and failed at least one chemotherapy regimen.

26. A therapeutic combination for use in treating soft tissue sarcoma, comprising: an anti-PD-L1 antibody, and anlotinib or a pharmaceutically acceptable salt thereof. wherein the anti-PD-L1 antibody comprises the following amino acid sequences: a heavy chain CDR1 region having at least 80% homology to an amino acid sequence set forth in SEQ ID NO: 1 or SEQ ID NO: 4; or a heavy chain CDR2 region having at least 80% homology to an amino acid sequence set forth in SEQ ID NO: 2 or SEQ ID NO: 5; or a heavy chain CDR3 region having at least 80% homology to an amino acid sequence set forth in SEQ ID NO: 3 or SEQ ID NO: 6; or a light chain CDR1 region having at least 80% homology to an amino acid sequence set forth in SEQ ID NO: 7 or SEQ ID NO: 10; or a light chain CDR2 region having at least 80% homology to an amino acid sequence set forth in SEQ ID NO: 8 or SEQ ID NO: 11; or a light chain CDR3 region having at least 80% homology to an amino acid sequence set forth in SEQ ID NO: 9 or SEQ ID NO: 12.

27. The therapeutic combination according to claim 26, comprising: a pharmaceutical composition of an anti-PD-L1 antibody, and a pharmaceutical composition of anlotinib or a pharmaceutically acceptable salt thereof.

28. The therapeutic combination according to claim 26 or 27, packaged in a kit further comprising instructions for combined use of the anti-PD-L1 antibody, and anlotinib or the pharmaceutically acceptable salt thereof in treating soft tissue sarcoma.

29. The therapeutic combination according to any one of claims 26-28, comprising: a pharmaceutical composition comprising 600-2400 mg of the anti-PD-L1 antibody and a pharmaceutical composition comprising 6 mg, 8 mg, 10 mg and/or 12 mg of anlotinib or the pharmaceutically acceptable salt thereof in a unit dose.

30. The therapeutic combination according to 29, comprising: a pharmaceutical composition comprising 600-2400 mg of the anti-PD-L1 antibody provided in multiple-dose form and a pharmaceutical composition comprising 6 mg, 8 mg, 10 mg and/or 12 mg of anlotinib or the pharmaceutically acceptable salt thereof in a unit dose.

31. The therapeutic combination according to any one of claims 26-30, wherein the pharmaceutical composition of the anti-PD-L1 antibody is a solution for injection; the pharmaceutical composition of anlotinib or the pharmaceutically acceptable salt thereof is an oral solid formulation.

32. The use according to any one of claims 1-13, or the method according to any one of claims 14-25, or the therapeutic combination according to any one of claims 26-31, wherein the anti-PD-L1 antibody comprises an amino acid sequence as follows: a heavy chain CDR1 region selected from the group consisting of SEQ ID NO: 1 and SEQ ID NO: 4; a heavy chain CDR2 region selected from the group consisting of SEQ ID NO: 2 and SEQ ID NO: 5; a heavy chain CDR3 region selected from the group consisting of SEQ ID NO: 3 and SEQ ID NO: 6; a light chain CDR1 region selected from the group consisting of SEQ ID NO: 7 and SEQ ID NO: 10; a light chain CDR2 region selected from the group consisting of SEQ ID NO: 8 and SEQ ID NO: 11; and a light chain CDR3 region selected from the group consisting of SEQ ID NO: 9 and SEQ ID NO: 12.

33. The use according to any one of claims 1-13, or the method according to any one of claims 14-25, or the therapeutic combination according to any one of claims 26-31, wherein the anti-PD-L1 antibody comprises: a heavy chain CDR1 region having an amino acid sequence set forth in SEQ ID NO: 1; a heavy chain CDR2 region having an amino acid sequence set forth in SEQ ID NO: 2; a heavy chain CDR3 region having an amino acid sequence set forth in SEQ ID NO: 3; a light chain CDR1 region having an amino acid sequence set forth in SEQ ID NO: 7; a light chain CDR2 region having an amino acid sequence set forth in SEQ ID NO: 8; and a light chain CDR3 region having an amino acid sequence set forth in SEQ ID NO: 9.

34. The use according to any one of claims 1-13, or the method according to any one of claims 14-25, or the therapeutic combination according to any one of claims 26-31, wherein the anti-PD-L1 antibody comprises the following amino acid sequences: a heavy chain variable region having at least 80% homology to an amino acid sequence set forth in SEQ ID NO: 13 or SEQ ID NO: 14; and a light chain variable region having at least 80% homology to an amino acid sequence set forth in SEQ ID NO: 15 or SEQ ID NO: 16.

35. The use according to any one of claims 1-13, or the method according to any one of claims 14-25, or the therapeutic combination according to any one of claims 26-31, wherein the anti-PD-L1 antibody comprises: a heavy chain variable region selected from the group consisting of heavy chain variable regions of humanized antibodies hu13C5-hIgG1, hu13C5-hIgG4, hu5G11-hIgG1 and hu5G11-hIgG4; and a light chain variable region selected from the group consisting of light chain variable regions of humanized antibodies hu13 C5-hIgG 1, hu13C5-hIgG4, hu5G11-hIgG1 and hu5G11-hIgG4.
